(19)

Europäisches
Patentamt

European
Patent Office

Office européen
des brevets

(11) **EP 4 321 621 A1**

(12) **EUROPEAN PATENT APPLICATION**
published in accordance with Art. 153(4) EPC

(43) Date of publication:
**14.02.2024 Bulletin 2024/07**

(21) Application number: **22784661.5**

(22) Date of filing: **04.04.2022**

(51) International Patent Classification (IPC):
*C12N 15/13* (2006.01)    *A61K 39/395* (2006.01)
*A61P 1/18* (2006.01)    *A61P 35/00* (2006.01)
*A61P 43/00* (2006.01)    *C07K 16/28* (2006.01)
*C07K 16/32* (2006.01)    *C07K 16/46* (2006.01)
*C12N 1/15* (2006.01)    *C12N 1/19* (2006.01)
*C12N 1/21* (2006.01)    *C12N 5/10* (2006.01)
*C12N 15/63* (2006.01)    *C12P 21/08* (2006.01)
*G01N 33/574* (2006.01)

(52) Cooperative Patent Classification (CPC):
A61K 39/395; A61P 1/18; A61P 35/00;
A61P 43/00; C07K 16/00; C07K 16/28;
C07K 16/32; C07K 16/46; C12N 5/10; C12N 15/63;
C12N 15/74; C12N 15/80; C12N 15/81;
G01N 33/574

(86) International application number:
**PCT/JP2022/017037**

(87) International publication number:
**WO 2022/215679 (13.10.2022 Gazette 2022/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.04.2021 JP 2021064090**

(71) Applicants:
• **University Public Corporation Osaka**
**Osaka-shi, Osaka 545-0051 (JP)**
• **National University Corporation Kobe University**
**Kobe-shi, Hyogo 657-8501 (JP)**

(72) Inventors:
• **TACHIBANA, Taro**
**Osaka-shi, Osaka 558-8585 (JP)**
• **IHARA, Kanichiro**
**Osaka-shi, Osaka 558-8585 (JP)**
• **HORI, Yuichi**
**Kobe-shi, Hyogo 657-8501 (JP)**
• **SHIMIZU, Kazuya**
**Kobe-shi, Hyogo 657-8501 (JP)**

(74) Representative: **Müller-Boré & Partner**
**Patentanwälte PartG mbB**
**Friedenheimer Brücke 21**
**80639 München (DE)**

Remarks:
The complete document including Reference
Table(s) and the Sequence Listing(s) can be
downloaded from the EPO website

(54) **ANTIBODY AGAINST PANCREATIC CANCER STEM CELLS**

(57)    The purpose of the present invention is to provide an antibody against pancreatic cancer stem cells. This antibody against pancreatic cancer stem cells includes a heavy chain variable region including heavy chain CDRs described as HCDR1-HCDR3, and a light chain variable region including light chain CDRs described as LCDR1-LCDR3, and has an ability to bind to pancreatic cancer stem cells, where HCDR1 has an amino acid sequence located at positions 26-35 in SEQ ID NO: 1 or a sequence analogous thereto, HCDR2 has a sequence located at positions 47-65 in SEQ ID NO: 1 or a sequence analogous thereto, HCDR3 has a sequence located at positions 96-104 in SEQ ID NO: 1 or a sequence analogous thereto, LCDR1 has a sequence located at positions 24-34 in SEQ ID NO: 2 or a sequence analogous thereto, LCDR2 has a sequence located at

EP 4 321 621 A1

**(Cont. next page)**

positions 46-56 in SEQ ID NO: 2 or a sequence analogous thereto, and LCDR3 has a sequence located at positions 89-96 in SEQ ID NO: 2 or a sequence analogous thereto.

**Description**

TECHNICAL FIELD

The present invention relates to an antibody against pancreatic cancer stem cells.

BACKGROUND ART

Cancer is the top cause of death for Japanese people and is the greatest threat for human beings. In cancer, pancreatic cancer results in a particularly poor prognosis, and about 70 to 80% of patients in which pancreatic cancer has been found have progressed to a state where surgery cannot be already performed. Furthermore, since the pancreas is surrounded by other organs, pancreatic cancer is likely to metastasize to the lymph nodes and the liver, and is difficult to treat. While improvement in surgical treatment technology and development of new drugs have progressed, the 5-year survival rate of pancreatic cancer patients is significantly lower than the average of those for all cancers, which indicates the difficulty of treatment.

Accordingly, improvement of the therapeutic effect on pancreatic cancer is an extremely serious challenge. Current cancer treatment includes surgical therapy including surgically removing a cancer tumor, chemotherapy using an anticancer agent, and radiation therapy. However, regarding the surgical therapy, a decrease in postoperative QOL of the patient is considered as a disadvantage. In addition, it has been revealed that chemotherapy and radiation therapy bring about resistance to cancer stem cells, and their mechanisms have been studied (Non-Patent Documents 1 to 5). Furthermore, a hypothesis that chemotherapy triggers reactivation of dormant cancer stem cells to induce recurrence of cancer tumors is being supported (Non-Patent Document 6).

Although there are many unknown parts in cancer stem cells, development of therapies targeting cancer stem cells has been variously advanced. For example, cancer stem cell markers or pharmaceutical products targeting signal pathways (Wnt, Notch) specifically activated in cancer stem cells have been developed (Non-Patent Document 7). In addition, gemtuzumab ozogamicin, an antibody-drug conjugate targeting the peripheral blood monocyte marker CD33, was approved by the FDA (U.S. Food and Drug Administration) for acute myeloid leukemia, but was discontinued because toxicity was found (Non-Patent Document 8).

PRIOR ART DOCUMENTS

NON-PATENT DOCUMENTS

Non-Patent Document 1: Borst P. (2012). Cancer drug pan-resistance: pumps, cancer stem cells, quiescence, epithelial to mesenchymal transition, blocked cell death pathways, persisters or what? Open Biol. 2, 120066.
Non-Patent Document 2: Holohan C., Van Schaeybroeck, S., Longley, D. B. & Johnston, P.G. (2013). Cancer drug resistance: an evolving paradigm. Nat. Rev. Cancer. 13, 714-726.
Non-Patent Document 3: Diehn M, Cho RW, Lobo NA, Kalisky T, Dorie MJ et al. (2009). Association of reactive oxygen species levels and radioresistance in cancer stem cells. Nature. 458, 780-783.
Non-Patent Document 4: Xiaoxian Li, Michael T. Lewis, Jian Huang, Carolina Gutierrez, C. Kent Osborne et al. (2008). Intrinsic resistance of tumorigenic breast cancer cells to chemotherapy. J. Natl. Cancer Inst. 100, 672-679.
Non-Patent Document 5: Shideng Bao, Qiulian Wu1, Roger E. McLendon, Yueling Hao, Qing Shi et al. (2006). Glioma stem cells promote radioresistance by preferential activation of the DNA damage response. Nature. 444, 756-760.
Non-Patent Document 6: Eduard Batlle & Hans Clevers (2017). Cancer stem cells revisited. Nat. Med. 23, 1124-1134.
Non-Patent Document 7: Naoko Takebe, Lucio Miele, Pamela Jo Harris, Woondong Jeong, Hideaki Bando et al. (2015). Targeting Notch, Hedgehog, and Wnt pathways in cancer stem cells: clinical update. Nat. Rev. Clin. Oncol. 12, 445-464.
Non-Patent Document 8: Laszlo G.S., Estey E.H. & Walter R.B. (2014). The past and future of CD33 as therapeutic target in acute myeloid leukemia. Blood Rev. 28, 143-153.

SUMMARY OF THE INVENTION

PROBLEMS TO BE SOLVED BY THE INVENTION

As there is a case where a pharmaceutical product is discontinued even when approved by the FDA, because toxicity is found, pharmaceutical products targeting cancer stem cells have a risk of affecting normal stem cells. Accordingly, pharmaceutical products are being developed but many of them remain at the stage of clinical trials. An object of the present invention is to provide an antibody against pancreatic cancer stem cells.

MEANS FOR SOLVING THE PROBLEM

The present inventor has succeeded in obtaining a new antibody having a binding property to pancreatic cancer stem cells by injecting a suspension prepared by mixing KMC07 cultured using a human pancreatic cancer stem cell line KMC07 established from a patient with advanced pancreatic ductal adenocarcinoma resistant to gemcitabine chemotherapy as an antigen and a mouse fibroblast cell line PA6 as a feeder cell with an adjuvant to provide cell immunization, and screening antibodies produced by hybridomas prepared and selected using lymphocytes collected after the immunization. The present invention has been completed by further conducting studies based on this finding.

In other words, the present invention provides an invention of the aspects described below.

Item 1. An antibody against pancreatic cancer stem cells, containing a heavy chain variable region including heavy chain CDRs as depicted in HCDR1 to HCDR3 below, and a light chain variable region including light chain CDRs as depicted in LCDR1 to LCDR3 below:

HCDR1 composed of an amino acid sequence from position 26 to position 35, from position 31 to position 35, from position 26 to position 33, or from position 27 to position 35 of SEQ ID NO: 1; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

HCDR2 composed of an amino acid sequence from position 47 to position 65, from position 50 to position 65, from position 51 to position 57, or from position 47 to position 59 of SEQ ID NO: 1; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

HCDR3 composed of an amino acid sequence from position 96 to position 104, from position 98 to position 104, or from position 97 to position 104 of SEQ ID NO: 1; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

LCDR1 composed of an amino acid sequence from position 24 to position 34, from position 27 to position 32, or from position 27 to position 34 of SEQ ID NO: 2; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

LCDR2 composed of an amino acid sequence from position 46 to position 56, from position 50 to position 56, or from position 50 to position 51 of SEQ ID NO: 2; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions, and

LCDR3 composed of an amino acid sequence from position 89 to position 96 or from position 89 to position 95 of SEQ ID NO: 2; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions.

Item 2. Antibody of item 1, in which the HCDR1 is composed of an amino acid sequence from position 26 to position 35, from position 31 to position 35, from position 26 to position 33, or from position 27 to position 35 of SEQ ID NO: 1 or SEQ ID NO: 3,

the HCDR2 is composed of an amino acid sequence from position 47 to position 65, from position 50 to position 65, from position 51 to position 57, from position 47 to position 59, or from position 47 to position 60 of SEQ ID NO: 1 or SEQ ID NO: 3,
the HCDR3 is composed of an amino acid sequence from position 96 to position 104, from position 98 to position 104, or from position 97 to position 104 of SEQ ID NO: 1 or SEQ ID NO: 3,
the LCDR1 is composed of an amino acid sequence from position 24 to position 34, from position 27 to position 32, or from position 27 to position 34 of SEQ ID NO: 2 or SEQ ID NO: 4,
the LCDR2 is composed of an amino acid sequence from position 46 to position 56, from position 50 to position 56, or from position 50 to position 51 of SEQ ID NO: 2 or SEQ ID NO: 4, and
the LCDR3 is composed of an amino acid sequence from position 89 to position 96 or from position 89 to position 95 of SEQ ID NO: 2 or SEQ ID NO: 4.

Item 3. The antibody of item 1 or 2, containing a heavy chain variable region and a light chain variable region below:

Heavy chain variable region composed of the amino acid sequence of SEQ ID NO: 1; an amino acid sequence having the amino acid sequence of SEQ ID NO: 1 in which one to several amino acids are substituted, deleted, added, and/or inserted; an amino acid sequence having 85% or more sequence identity to the amino acid sequence of SEQ ID NO: 1; or an amino acid sequence encoded by a polynucleotide that specifically hybridizes to a polynucleotide composed of a base sequence complementary to a base sequence encoding the amino acid sequence of SEQ ID NO: 1 under stringent conditions, and
light chain variable region composed of the amino acid sequence of SEQ ID NO: 2; an amino acid sequence having the amino acid sequence of SEQ ID NO: 2 in which one to several amino acids are substituted, deleted, added, and/or inserted; an amino acid sequence having 85% or more sequence identity to the amino acid sequence of SEQ ID NO: 2; or an amino acid sequence encoded by a polynucleotide that specifically hybridizes to a polynucleotide composed of a base sequence complementary to a base sequence encoding the amino acid sequence of SEQ ID NO: 2 under stringent conditions.

Item 4. The antibody of item 3, in which the heavy chain variable region is composed of the amino acid sequence of SEQ ID NO: 1 or SEQ ID NO: 3, and the light chain variable region is composed of the amino acid sequence of SEQ ID NO: 2 or SEQ ID NO: 4.

Item 5. An antibody against pancreatic cancer stem cells, containing a heavy chain variable region including heavy chain CDRs as depicted in HCDR1 to HCDR3 below, and a light chain variable region including light chain CDRs as depicted in LCDR1 to LCDR3 below:

HCDR1 composed of an amino acid sequence from position 26 to position 35, from position 31 to position 35, from position 26 to position 33, or from position 27 to position 35 of SEQ ID NO: 5; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,
HCDR2 composed of an amino acid sequence from position 47 to position 66, from position 50 to position 66, from position 51 to position 58, or from position 47 to position 60 of SEQ ID NO: 5; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,
HCDR3 composed of an amino acid sequence from position 97 to position 111, from position 99 to position 111, or from position 98 to position 111 of SEQ ID NO: 5; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,
LCDR1 composed of an amino acid sequence from position 24 to position 34, from position 27 to position 32, or from position 27 to position 34 of SEQ ID NO: 6; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

LCDR2 composed of an amino acid sequence from position 46 to position 56, from position 50 to position 56, or from position 50 to position 51 of SEQ ID NO: 6; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions, and

LCDR3 composed of an amino acid sequence from position 89 to position 98 or from position 89 to position 97 of SEQ ID NO: 6; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions.

Item 6. An anti-EpCAM antibody, containing a heavy chain variable region including heavy chain CDRs as depicted in HCDR1 to HCDR3 below, and a light chain variable region including light chain CDRs as depicted in LCDR1 to LCDR3 below:

HCDR1 composed of an amino acid sequence from position 26 to position 35, from position 31 to position 35, from position 26 to position 33, or from position 27 to position 35 of SEQ ID NO: 7; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

HCDR2 composed of an amino acid sequence from position 47 to position 66, from position 50 to position 66, from position 51 to position 58, or from position 47 to position 61 of SEQ ID NO: 7; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

HCDR3 composed of an amino acid sequence from position 97 to position 112, from position 99 to position 112, or from position 98 to position 112 of SEQ ID NO: 7; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

LCDR1 composed of an amino acid sequence from position 24 to position 34, from position 27 to position 32, or from position 27 to position 34 of SEQ ID NO: 8; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

LCDR2 composed of an amino acid sequence from position 46 to position 56, from position 50 to position 56, or from position 50 to position 51 of SEQ ID NO: 8; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions, and

LCDR3 composed of an amino acid sequence from position 89 to position 97 or from position 89 to position 96 of SEQ ID NO: 8; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions.

Item 7. The antibody of any of items 1 to 6, which is a monoclonal antibody.
Item 8. The antibody of any of items 1 to 7, which is a human chimerized antibody or a mouse chimerized antibody.
Item 9. A DNA encoding the antibody of any of items 1 to 8.
Item 10. A recombinant vector containing the DNA of item 9.
Item 11. The recombinant vector of item 10, containing a light chain signal sequence composed of a base sequence set forth in SEQ ID NO: 20, 24, or 28.
Item 12. A transformant into which the recombinant vector of item 10 or item 11 has been introduced.
Item 13. A method for producing an antibody, including the step of culturing the transformant of item 12 to produce the antibody of any of items 1 to 8.
Item 14. A pharmaceutical composition containing the antibody of any of items 1 to 8 as an active ingredient.
Item 15. The pharmaceutical composition of item 14 for use in treatment of pancreatic cancer.
Item 16. An extracorporeal diagnostic agent composed of the antibody of any of items 1 to 8.
Item 17. The extracorporeal diagnostic agent of item 16 for use in detection of extracellular vesicles derived from cancer cells.
Item 18. The extracorporeal diagnostic agent of item 16 or 17 for use in diagnosis of pancreatic cancer.
Item 19. An antibody substrate containing the extracorporeal diagnostic agent of any of items 16 to 18, and a substrate on which the extracorporeal diagnostic agent is immobilized.
Item 20. An extracorporeal diagnostic composition containing the extracorporeal diagnostic agent of any of items 16 to 18.

ADVANTAGES OF THE INVENTION

According to the present invention, a new antibody having a binding property to pancreatic cancer stem cells can be provided.

BRIEF DESCRIPTION OF THE DRAWINGS

Fig. 1 shows a flow of antibody production performed in Test Example 1.
Fig. 2 shows a flow of antibody gene cloning performed in Test Example 2.
Fig. 3 shows the result of agarose electrophoresis of a variable region cloned in Test Example 2.
Fig. 4 shows the result of immunostaining of an anti-KMC07 antibody expressed in Test Example 2.
Fig. 5 shows a flow of construction of a signal sequence 1E7SP recombinant light chain expression vector performed in Test Example 2.
Fig. 6 shows the result of immunostaining of an anti-KMC07 antibody expressed by signal sequence 1E7SP recombination in Test Example 2.
Fig. 7 shows a flow of construction of a signal sequence 1B4SP recombinant light chain expression vector performed in Test Example 2.
Fig. 8A shows a comparison of the antibody secretion amount by dot blot of an anti-KMC07 antibody (rat antibody) expressed by signal sequence 1B4SP recombination in Test Example 2.
Fig. 8B shows a comparison of the antibody secretion amount by dot blot of the anti-KMC07 antibody (mouse chimerized antibody) expressed by signal sequence 1B4SP recombination in Test Example 2.
Fig. 9 shows the result of immunostaining of KMC07 using antibodies (a: rat antibody, b: rat/human chimerized antibody, c: rat/mouse chimerized antibody) prepared in Test Example 2.
Fig. 10 shows the result of silver staining of immunoprecipitates using KMC07 performed in Test Example 3.
Fig. 11 shows the result of silver staining of an extract prepared using an anti-KMC07 antibody-binding column in Test Example 3.
Fig. 12 shows the result of living cell immunostaining using KMC07 performed in Test Example 3.
Fig. 13 shows the ADCC activity of an anti-KMC07 antibody (1F9D4: antibody III) against pancreatic cancer cells, confirmed in Test Example 3.
Fig. 14A shows the in vitro evaluation result of the ADCC activity of an anti-KMC07 antibody (1B4G: antibody I) against KMC07 confirmed in Test Example 3, showing the number of colonies of KMC07 at each reaction time (data are shown as mean $\pm$ standard error (n = 3)) (*P < 0.05, **P < 0.001, two-way ANOVA).
Fig. 14B shows the in vitro evaluation result of the ADCC activity of an anti-KMC07 antibody (1F9D4: antibody III) against KMC07 confirmed in Test Example 3, showing the number of colonies of KMC07 at each reaction time (data are shown as mean $\pm$ standard error (n = 3)) (*P < 0.05, **P < 0.001, two-way ANOVA).

Fig. 15 shows the immunostained image of KMC07 24 hours after reaction with 1F9D4 (antibody III) obtained in Test Example 3.

Fig. 16A shows the temporal change of the rate of weight gain/loss based on the start date of the clinical trial of anti-KMC07 mouse chimeric antibodies 1E7G5 (antibody I) and 1F9D4 (antibody III) confirmed in Test Example 3, in which the red arrow indicates antibody administration, and the data are shown as mean $\pm$ standard error (n = 3).

Fig. 16B shows the temporal change of the tumor volume ratio $V/V_0$ based on the start date of the clinical trial of anti-KMC07 mouse chimeric antibodies 1E7G5 (antibody I) and 1F9D4 (antibody III) confirmed in Test Example 3, in which the red arrow indicates antibody administration, and the data are shown as mean $\pm$ standard error (n = 3) (P < 0.05, **P < 0.001, vs. 4G8B4, two-way ANOVA).

Fig. 17 shows an evaluation of reactivity of anti-KMC07 antibodies 1B4G4 (antibody I), 1E7G5 (antibody I), 1E9F7 (antibody II), and 1F9D4 (antibody III) against KMC07-derived extracellular vesicles by dot blot confirmed in Test Example 4.

Fig. 18 shows a schematic diagram (a) of a sandwich ELISA experimental system used in Test Example 4, and an evaluation of reactivity of anti-KMC07 antibodies 1B4G4 (antibody I), 1E7G5 (antibody I), 1E9F7 (antibody II), and 1F9D4 (antibody III) against KMC07-derived extracellular vesicles by sandwich ELISA.

Fig. 19 shows a schematic diagram (a) of a fluorescence detection sandwich ELISA experimental system used in Test Example 4, and an evaluation of reactivity of anti-KMC07 antibodies 1B4G4 (antibody I), 1E7G5 (antibody I), 1E9F7 (antibody II), and 1F9D4 (antibody III) against a KMC07-derived exosome in a cell culture supernatant.

EMBODIMENTS OF THE INVENTION

1. Antibodies against pancreatic cancer stem cells

The present invention is an antibody against pancreatic cancer stem cells. The antibody against pancreatic cancer stem cells refers to an antibody having at least a binding property to pancreatic cancer stem cells, and the binding property at least needs to have binding ability to pancreatic cancer stem cells but not have binding ability to normal cells, which binding property includes both of a binding property specific to pancreatic cancer stem cells and a binding property not specific to pancreatic cancer stem cells (in other words, a property of binding not only to pancreatic cancer but also to cancer cells other than pancreatic cancer). The antibody of the present invention includes the following antibody I, antibody II, and antibody III.

The CDRs (complementarity determining regions) of the antibody of the present invention include CDRs defined by all combinations (hereinafter, also referred to as "Kabat/IMGT/Paratome".) of the Kabat numbering program (Bioinformatics, 32, 298-300 (2016)), the IMGT system (Dev Comp Immunol. 27, 55-77 (2003)), and the Paratome algorithm (Nucleic Acids Res. 40 (Web Server issue): W521-4 (2012). doi: 10.1093/nar/gks 480 and PLoS Comput Biol. 8(2): e 1002388 (2012)), and CDRs defined by any of the Kabat numbering program, the IMGT system, and the Paratome algorithm. The sequences of CDRs as defined by Kabat/IMGT/Paratome encompass all of the sequences of CDRs as defined by the Kabat numbering program, the sequences of CDRs as defined by the IMGT system, and the sequences of CDRs as defined by the Paratome algorithm.

<Antibody I>

Antibody I is an antibody against pancreatic cancer stem cells containing a heavy chain variable region including heavy chain CDRs as depicted in (1-1) HCDR1 to (1-3) HCDR3 below, and a light chain variable region including light chain CDRs as depicted in (I-4) LCDR1 to (1-6) LCDR3 below.

In the following, SEQ ID NO: 1 represents an amino acid sequence of the heavy chain variable region of an example of antibody I (derived from rat), and SEQ ID NO: 2 represents an amino acid sequence of the light chain variable region of an example of antibody I (derived from rat).

(1-1) HCDR1 composed of an amino acid sequence from position 26 to position 35, from position 31 to position 35, from position 26 to position 33, or from position 27 to position 35 of SEQ ID NO: 1; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

(1-2) HCDR2 composed of an amino acid sequence from position 47 to position 65, from position 50 to position 65, from position 51 to position 57, or from position 47 to position 59 of SEQ ID NO: 1; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

(1-3) HCDR3 composed of an amino acid sequence from position 96 to position 104, from position 98 to position 104, or from position 97 to position 104 of SEQ ID NO: 1; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

(I-4) LCDR1 composed of an amino acid sequence from position 24 to position 34, from position 27 to position 32, or from position 27 to position 34 of SEQ ID NO: 2; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

(I-5) LCDR2 composed of an amino acid sequence from position 46 to position 56, from position 50 to position 56, or from position 50 to position 51 of SEQ ID NO: 2; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions, and

(I-6) LCDR3 composed of an amino acid sequence from position 89 to position 96 or from position 89 to position 95 of SEQ ID NO: 2; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions.

In the above (1-1) to (I-6), an antibody containing a heavy chain and/or light chain complementarity determining region (hereinafter also collectively referred to as "CDR") composed of the amino acid sequence in which one to several amino acids are substituted, deleted, added, and/or inserted only needs to have a binding property to pancreatic cancer stem cells. The number of amino acids to be substituted, deleted, added, and/or inserted is not particularly limited, but is preferably 1 to 3, more preferably 1 to 2, particularly preferably 1 per CDR. In the antibody of the present invention, substitution, deletion, addition, and/or insertion may be made in the amino acid sequence of one CDR, or substitution, deletion, addition, and/or insertion may be made in the amino acid sequence of two or more CDRs in the CDR sequence.

In addition, with respect to amino acid substitutions in the amino acid sequences of CDRs, substitutions with similar amino acids (that is, conservative amino acid substitutions), except for the 52nd position of SEQ ID NO: 1, are suitable because it is predicted that these substitutions do not adversely affect a binding property to pancreatic cancer stem cells. Specifically, the following grouping has been established based on the properties of amino acid side chains, and amino acids belonging to the same group are similar to each other.

Basic amino acids: lysine, arginine, histidine
Acidic amino acids: glutamic acid, aspartic acid
Neutral amino acids: glycine, alanine, serine, threonine, methionine, cysteine, phenylalanine, tryptophan, tyrosine, leucine, isoleucine, valine, glutamine, asparagine, proline

Furthermore, the neutral amino acids can also be grouped into those having a polar side chain (asparagine, glutamine, serine, threonine, tyrosine, cysteine), those having a non-polar side chain (glycine, alanine, valine, leucine, isoleucine, proline, phenylalanine, methionine, tryptophan), those having an amide-containing side chain (asparagine, glutamine), those having a sulfur-containing side chain (methionine, cysteine), those having an aromatic side chain (phenylalanine, tryptophan, tyrosine), those having a hydroxyl group-containing side chain (serine, threonine, tyrosine), those having an aliphatic side chain (alanine, leucine, isoleucine, valine), and the like. The 52nd amino acid residue (aspartic acid residue) of SEQ ID NO: 1 can be substituted with an amino acid other than the similar amino acid, for example, an asparagine residue.

As a method for substituting one or several amino acid residues with another amino acid of interest, for example, a site-directed mutagenesis method (Hashimoto-Gotoh T. et al., Gene, Vol. 152, p. 271-275 (1995); Zoller MJ. et al., Methods Enzymol. Vol. 100, p. 468-500 (1983); Kramer W. et al., Nucleic Acids Res. Vol. 12, p. 9441-9456 (1984);

Kramer W. et al., Methods. Enzymol. Vol. 154, p. 350-367 (1987); Kunkel TA., Proc Natl Acad Sci USA., Vol. 82, p. 488-492 (1985), etc.) is known, and amino acid substitutions can be made on the amino acid sequence of CDRs using the site-directed mutagenesis method. The method of substitution with another amino acid includes the library technique described in WO 2005/080432.

In the above (1-1) to (I-6), an antibody containing CDRs each composed of the amino acid sequence having 85% or more sequence identity only needs to have a binding property to pancreatic cancer stem cells. The sequence identity only needs to be 85% or more, but is preferably 87% or more, more preferably 92% or more, 95% or more, 96% or more, 97% or more, or 98% or more, particularly preferably 99% or more. Here, for example, the sequence identity to a predetermined amino acid sequence is sequence identity calculated by comparing with the predetermined amino acid sequence.

In addition, the "sequence identity" indicates a value of identity of an amino acid sequence obtained by bl2seq program (Tatiana A. Tatsusova, Thomas L. Madden, FEMS Microbiol. Lett., Vol. 174, p247-250, 1999) of BLAST PACKAGE [sgi32 bit edition, Version 2.0.12; available from National Center for Biotechnology Information (NCBI)]. The parameters may be set to Gap insertion Cost value: 11 and Gap extension Cost value: 1.

In the above (1-1) to (I-6), the "under stringent conditions" refer to conditions of incubation at 50°C to 65°C for 4 hours to overnight in 6 × SSC (1× SSC is 0.15 M NaCl, 0.015 M sodium citrate, pH 7.0) containing 0.5% SDS, 5× Denhartz's [0.1% Bovine Serum Albumin (BSA), 0.1% Polyvinylpyrrolidone, 0.1% Ficoll 400], and 100 μg/ml salmon sperm DNA.

Hybridization under stringent conditions is specifically performed by the following technique. That is, a nylon membrane on which a DNA library or a cDNA library is immobilized is prepared, and the nylon membrane is blocked at 65°C in a prehybridization solution containing 6× SSC, 0.5% SDS, 5× Denhartz's, and 100 μg/ml salmon sperm DNA. Each probe labeled $^{32}$P is then added, followed by incubation at 65°C overnight. This nylon membrane is washed in 6× SSC for 10 minutes at room temperature, in 2× SSC containing 0.1% SDS for 10 minutes at room temperature, and in 0.2× SSC containing 0.1% SDS for 30 minutes at 45°C, followed by autoradiography, by which DNA specifically hybridized with the probe can be detected.

<Suitable examples of antibody I>

Suitable examples of antibody I of the present invention include antibodies containing a heavy chain variable region including heavy chain CDRs as depicted in (I-1') HCDR1 to (I-3') HCDR3 below, and a light chain variable region including light chain CDRs as depicted in (I-4') LCDR1 to (I-6') LCDR3 below.

In the following, SEQ ID NO: 3 represents an amino acid sequence of the heavy chain variable region of another example of antibody I (derived from rat), and SEQ ID NO: 4 represents an amino acid sequence of the light chain variable region of another example of antibody I (derived from rat). SEQ ID NO: 3 represents an amino acid sequence of the amino acid sequence of SEQ ID NO: 1 in which the 60th amino acid residue (asparagine residue) is substituted with a cysteine residue and the 62nd amino acid residue (alanine residue) is substituted with a proline residue. SEQ ID NO: 4 is an amino acid sequence of the amino acid sequence of SEQ ID NO: 2 in which the 32nd amino acid residue (isoleucine residue) is substituted with an asparagine residue, the 46th amino acid residue (leucine residue) is substituted with a phenylalanine residue, and the 52nd amino acid residue (aspartic acid residue) is substituted with an asparagine residue.

(I-1') the (I-1) HCDR1 is composed of an amino acid sequence from position 26 to position 35, from position 31 to position 35, from position 26 to position 33, or from position 27 to position 35 of SEQ ID NO: 1 or SEQ ID NO: 3,
(I-2') the (I-2) HCDR2 is composed of an amino acid sequence from position 47 to position 65, from position 50 to position 65, from position 51 to position 57, from position 47 to position 59, or from position 47 to position 60 of SEQ ID NO: 1 or SEQ ID NO: 3,
(I-3') the (I-3) HCDR3 is composed of an amino acid sequence from position 96 to position 104, from position 98 to position 104, or from position 97 to position 104 of SEQ ID NO: 1 or SEQ ID NO: 3,
(I-4') the (I-4) LCDR1 is composed of an amino acid sequence from position 24 to position 34, from position 27 to position 32, or from position 27 to position 34 of SEQ ID NO: 2 or SEQ ID NO: 4,
(I-5') the (I-5) LCDR2 is composed of an amino acid sequence from position 46 to position 56, from position 50 to position 56, or from position 50 to position 51 of SEQ ID NO: 2 or SEQ ID NO: 4,
(I-6') the (I-6) LCDR3 is composed of an amino acid sequence from position 89 to position 96 or from position 89 to position 95 of SEQ ID NO: 2 or SEQ ID NO: 4.

<Another suitable example of antibody I>

In antibody I of the present invention, the framework regions of the heavy chain variable region and the light chain variable region are not particularly limited as long as they do not substantially affect the binding activity to pancreatic cancer stem cells. Another suitable example of antibody I of the present invention includes antibodies containing a heavy chain variable region and a light chain variable region below.

Heavy chain variable region composed of the amino acid sequence of SEQ ID NO: 1; an amino acid sequence having the amino acid sequence of SEQ ID NO: 1 in which one to several amino acids are substituted, deleted, added, and/or inserted; an amino acid sequence having 85% or more sequence identity to the amino acid sequence of SEQ ID NO: 1; or an amino acid sequence encoded by a polynucleotide that specifically hybridizes to a polynucleotide composed of a base sequence complementary to a base sequence encoding the amino acid sequence of SEQ ID NO: 1 under stringent conditions, and

light chain variable region composed of the amino acid sequence of SEQ ID NO: 2; an amino acid sequence having the amino acid sequence of SEQ ID NO: 2 in which one to several amino acids are substituted, deleted, added, and/or inserted; an amino acid sequence having 85% or more sequence identity to the amino acid sequence of SEQ ID NO: 2; or an amino acid sequence encoded by a polynucleotide that specifically hybridizes to a polynucleotide composed of a base sequence complementary to a base sequence encoding the amino acid sequence of SEQ ID NO: 2 under stringent conditions.

In another suitable example of the above antibody I, an antibody containing a heavy chain and/or light chain variable region (hereinafter, also collectively referred to as a "variable region") composed of the amino acid sequence in which one to several amino acids are substituted, deleted, added, and/or inserted only needs to have a binding property to pancreatic cancer stem cells. The number of amino acids to be substituted, deleted, added, and/or inserted is not particularly limited, but is preferably 1 to 15, more preferably 1 to 10, still more preferably 1 to 5, still more preferably 1 to 4, still more preferably 1 to 3, particularly preferably 1 to 2, most preferably 1 for each variable region. Details of the matter related to amino acid substitution (conservative amino acid substitution, method of substitution) are the same as those described in (1-1) to (I-6) above.

In another suitable example of the above antibody I, an antibody containing variable regions each composed of the amino acid sequence having 85% or more sequence identity only needs to have a binding property to pancreatic cancer stem cells. Details of sequence identity and stringent conditions are the same as those described in (I-1) to (I-6) above.

<Particularly suitable example of antibody I>

Particularly suitable examples of antibody I of the present invention include antibodies containing a heavy chain variable region composed of the amino acid sequence of SEQ ID NO: 1 or SEQ ID NO: 3 and a light chain variable region composed of the amino acid sequence of SEQ ID NO: 2 or SEQ ID NO: 4.

<Antibody II>

Antibody II is an antibody against pancreatic cancer stem cells containing a heavy chain variable region including heavy chain CDRs as depicted in (II-1) HCDR1 to (II-3) HCDR3 below, and a light chain variable region including light chain CDRs as depicted in (II-4) LCDR1 to (II-6) LCDR3 below.

(II-1) HCDR1 composed of an amino acid sequence from position 26 to position 35, from position 31 to position 35, from position 26 to position 33, or from position 27 to position 35 of SEQ ID NO: 5; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

(II-2) HCDR2 composed of an amino acid sequence from position 47 to position 66, from position 50 to position 66, from position 51 to position 58, or from position 47 to position 60 of SEQ ID NO: 5; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

(II-3) HCDR3 composed of an amino acid sequence from position 97 to position 111, from position 99 to position 111, or from position 98 to position 111 of SEQ ID NO: 5; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

(II-4) LCDR1 composed of an amino acid sequence from position 24 to position 34, from position 27 to position 32, or from position 27 to position 34 of SEQ ID NO: 6; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,

(II-5) LCDR2 composed of an amino acid sequence from position 46 to position 56, from position 50 to position 56, or from position 50 to position 51 of SEQ ID NO: 6; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions, and

(II-6) LCDR3 composed of an amino acid sequence from position 89 to position 98 or from position 89 to position 97 of SEQ ID NO: 6; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions.

In the above (II-1) to (II-6), an antibody containing CDRs each composed of the amino acid sequence in which one to several amino acids are substituted, deleted, added, and/or inserted only needs to have a binding property to pancreatic cancer stem cells. Details of the number of amino acids to be substituted, deleted, added, and/or inserted and the like are the same as those described in (1-1) to (I-6) of antibody I above. With respect to amino acid substitutions in the amino acid sequences of the CDRs, substitutions with similar amino acids (that is, conservative amino acid substitutions) are suitable because it is predicted that these substitutions do not adversely affect a binding property to pancreatic cancer stem cells. Details of the amino acid substitution are the same as those described in (1-1) to (I-6) of antibody I above.

In the above (II-1) to (II-6), an antibody containing CDRs each composed of the amino acid sequence having 85% or more sequence identity only needs to have a binding property to pancreatic cancer stem cells. Details of sequence identity and stringent conditions are the same as those described in (1-1) to (I-6) related to antibody I above.

<Suitable examples of antibody II>

In antibody II of the present invention, the framework regions of the variable region are not particularly limited as long as they do not substantially affect the binding activity to pancreatic cancer stem cells. Accordingly, suitable examples of antibody II of the present invention includes antibodies containing a heavy chain variable region and a light chain variable region below.

In the following, SEQ ID NO: 5 represents an amino acid sequence of the heavy chain variable region of an example of antibody II (derived from rat), and SEQ ID NO: 6 represents an amino acid sequence of the light chain variable region of an example of antibody II (derived from rat).

Heavy chain variable region composed of the amino acid sequence of SEQ ID NO: 5; an amino acid sequence having the amino acid sequence of SEQ ID NO: 5 in which one to several amino acids are substituted, deleted, added, and/or inserted; an amino acid sequence having 85% or more sequence identity to the amino acid sequence of SEQ ID NO: 5; or an amino acid sequence encoded by a polynucleotide that specifically hybridizes to a polynucleotide composed of a base sequence complementary to a base sequence encoding the amino acid sequence of SEQ ID NO: 5 under stringent conditions, and

light chain variable region composed of the amino acid sequence of SEQ ID NO: 6; an amino acid sequence having the amino acid sequence of SEQ ID NO: 6 in which one to several amino acids are substituted, deleted, added, and/or inserted; an amino acid sequence having 85% or more sequence identity to the amino acid sequence of SEQ ID NO: 6; or an amino acid sequence encoded by a polynucleotide that specifically hybridizes to a polynucleotide composed of a base sequence complementary to a base sequence encoding the amino acid sequence of SEQ ID NO: 6 under stringent conditions.

In suitable examples of the above antibody II, an antibody containing a variable region composed of the amino acid sequence in which one to several amino acids are substituted, deleted, added, and/or inserted only needs to have a binding property to pancreatic cancer stem cells. Details of the number of amino acids to be substituted, deleted, added, and/or inserted are the same as those described in the other suitable examples of antibody I above, and details of the matter related to amino acid substitution (conservative amino acid substitution, method of substitution) are the same as those described in (1-1) to (I-6) related to antibody I above.

In suitable examples of the above antibody II, an antibody containing variable regions each composed of the amino acid sequence having 85% or more sequence identity only needs to have a binding property to pancreatic cancer stem cells. Details of sequence identity and stringent conditions are the same as those described in (1-1) to (I-6) related to antibody I above.

<Particularly suitable example of antibody II>

Particularly preferred examples of antibody II of the present invention include antibodies containing a heavy chain variable region composed of the amino acid sequence of SEQ ID NO: 5 and a light chain variable region composed of the amino acid sequence of SEQ ID NO: 6.

<Antibody III>

Antibody III is an anti-EpCAM antibody containing a heavy chain variable region including heavy chain CDRs as depicted in (III-1) HCDR1 to (III-3) HCDR3 below, and a light chain variable region including light chain CDRs as depicted in (III-4) LCDR1 to (III-6) LCDR3 below. EpCAM (epithelial cell adhesion molecule) is a transmembrane glycoprotein composed of 314 amino acids present on a cell membrane surface, and is overexpressed in various cancer tissues in addition to pancreatic cancer stem cells. Accordingly, antibody III is also an antibody against pancreatic cancer stem cells.

(III-1) HCDR1 composed of an amino acid sequence from position 26 to position 35, from position 31 to position 35, from position 26 to position 33, or from position 27 to position 35 of SEQ ID NO: 7; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,
(III-2) HCDR2 composed of an amino acid sequence from position 47 to position 66, from position 50 to position 66, from position 51 to position 58, or from position 47 to position 61 of SEQ ID NO: 7; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,
(III-3) HCDR3 composed of an amino acid sequence from position 97 to position 112, from position 99 to position 112, or from position 98 to position 112 of SEQ ID NO: 7; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,
(III-4) LCDR1 composed of an amino acid sequence from position 24 to position 34, from position 27 to position 32, or from position 27 to position 34 of SEQ ID NO: 8; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions,
(III-5) LCDR2 composed of an amino acid sequence from position 46 to position 56, from position 50 to position 56, or from position 50 to position 51 of SEQ ID NO: 8; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions, and
(III-6) LCDR3 composed of an amino acid sequence from position 89 to position 97 or from position 89 to position 96 of SEQ ID NO: 8; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide composed of a base sequence complementary to a base sequence encoding the amino acid sequence under stringent conditions.

In the above (III-I) to (III-6), an antibody containing CDRs each composed of the amino acid sequence in which one to several amino acids are substituted, deleted, added, and/or inserted only needs to have a binding property to pancreatic cancer stem cells. Details of the number of amino acids to be substituted, deleted, added, and/or inserted and the like are the same as those described in (1-1) to (I-6) of antibody I above. With respect to amino acid substitutions in the amino acid sequences of the CDRs, substitutions with similar amino acids (that is, conservative amino acid substitutions) are suitable because it is predicted that these substitutions do not adversely affect a binding property to pancreatic cancer stem cells. Details of the amino acid substitution are the same as those described in (1-1) to (I-6) of antibody I above.

In the above (III-I) to (III-6), an antibody containing CDRs each composed of the amino acid sequence having 85% or more sequence identity only needs to a have binding property to pancreatic cancer stem cells. Details of sequence identity and stringent conditions are the same as those described in (1-1) to (I-6) related to antibody I above.

<Suitable examples of antibody III>

In antibody III of the present invention, the framework regions of the variable region are not particularly limited as long as they do not substantially affect the binding activity to pancreatic cancer stem cells. Accordingly, suitable examples of antibody III of the present invention includes antibodies containing a heavy chain variable region and a light chain variable region below.

In the following, SEQ ID NO: 7 represents an amino acid sequence of the heavy chain variable region of an example of antibody III (derived from rat), and SEQ ID NO: 8 represents an amino acid sequence of the light chain variable region of an example of antibody III (derived from rat).

Heavy chain variable region composed of the amino acid sequence of SEQ ID NO: 7; an amino acid sequence having the amino acid sequence of SEQ ID NO: 7 in which one to several amino acids are substituted, deleted, added, and/or inserted; an amino acid sequence having 85% or more sequence identity to the amino acid sequence of SEQ ID NO: 7; or an amino acid sequence encoded by a polynucleotide that specifically hybridizes to a polynucleotide composed of a base sequence complementary to a base sequence encoding the amino acid sequence of SEQ ID NO: 7 under stringent conditions, and

light chain variable region composed of the amino acid sequence of SEQ ID NO: 8; an amino acid sequence having the amino acid sequence of SEQ ID NO: 6 in which one to several amino acids are substituted, deleted, added, and/or inserted; an amino acid sequence having 85% or more sequence identity to the amino acid sequence of SEQ ID NO: 8; or an amino acid sequence encoded by a polynucleotide that specifically hybridizes to a polynucleotide composed of a base sequence complementary to a base sequence encoding the amino acid sequence of SEQ ID NO: 8 under stringent conditions.

In suitable examples of the above antibody III, an antibody containing a heavy chain and/or light chain variable region composed of the amino acid sequence in which one to several amino acids are substituted, deleted, added, and/or inserted only needs to have a binding property to pancreatic cancer stem cells. Details of the number of amino acids to be substituted, deleted, added, and/or inserted are the same as those described in the other suitable examples of antibody I above, and details of the matter related to amino acid substitution (conservative amino acid substitution, method of substitution) are the same as those described in (1-1) to (I-6) related to antibody I above.

In suitable examples of the above antibody III, an antibody containing variable regions each composed of the amino acid sequence having 85% or more sequence identity only needs to have a binding property to pancreatic cancer stem cells. Details of sequence identity and stringent conditions are the same as those described in (1-1) to (I-6) related to antibody I above.

<Particularly suitable example of antibody III>

Particularly preferred examples of antibody III of the present invention include antibodies containing a heavy chain variable region composed of the amino acid sequence of SEQ ID NO: 7 and a light chain variable region composed of the amino acid sequence of SEQ ID NO: 8.

<Constant region>

In the antibody of the present invention, the amino acid sequence of the constant region is also not particularly limited as long as it does not substantially affect the binding activity to pancreatic cancer stem cells.

The amino acid sequences of the variable regions set forth in SEQ ID NOs: 1 to 8 are derived from rats. Accordingly, examples of the antibody of the present invention include a rat antibody having a rat-derived constant region.

<Signal region>

The antibody of the present invention can contain any signal region. For example, examples of the sequence of the heavy chain signal region include amino acid sequences set forth in SEQ ID NO: 17 or 21, SEQ ID NO: 25, and SEQ ID NO: 29, and examples of the sequence of the light chain signal region include amino acid sequences set forth in SEQ ID NO: 19 or 23, and SEQ ID NO: 27.

Regarding the signal region contained in antibody I, examples of the sequence of the heavy chain signal region include an amino acid sequence set forth in SEQ ID NO: 17 or 21, and examples of the sequence of the light chain signal region include an amino acid sequence set forth in SEQ ID NO: 19 or 23, preferably an amino acid sequence set forth in SEQ ID NO: 19. Regarding the signal region contained in antibody II, examples of the sequence of the heavy chain signal region include an amino acid sequence set forth in SEQ ID NO: 25, and examples of the sequence of the light chain signal region include an amino acid sequence set forth in SEQ ID NO: 27. Regarding the signal region contained in antibody III, examples of the sequence of the heavy chain signal region include an amino acid sequence set forth in SEQ ID NO: 29, and examples of the sequence of the light chain signal region include an amino acid sequence set forth in SEQ ID NO: 19.

<Recombinant antibody>

On the other hand, preferred examples of the antibody of the present invention include chimeric antibodies (mouse chimerized antibody, human chimerized antibody, etc.) and humanized antibodies.

The chimeric antibody is an antibody in which regions whose origins are different from each other are linked to each other. When used as an active ingredient of a therapeutic pharmaceutical composition, the chimeric antibody is preferably a rat-human chimeric antibody (human chimerized antibody) composed of a variable region derived from a rat antibody and a constant region derived from a human antibody. In addition, when used as a diagnostic agent, the chimeric antibody is preferably a rat-human chimeric antibody (human chimerized antibody) or a rat-mouse chimeric antibody (mouse chimerized antibody) composed of a variable region derived from a rat antibody and a constant region derived from a mouse antibody.

In addition, the humanized antibody is obtained by grafting a non-human-derived CDR sequence onto a framework region of a human antibody, and is an antibody composed of a CDR of a non-human-derived antibody, a framework region derived from a human antibody, and a constant region derived from a human antibody. Since the humanized antibody has reduced antigenicity in the human body, the antibody of the present invention is suitable when used as an active ingredient of a therapeutic pharmaceutical composition.

<Isotype>

The isotype of the antibody of the present invention is not particularly limited, but examples thereof include IgG (specific examples thereof include IgG1, IgG2, IgG3, and IgG4), IgA (specific examples thereof include IgA1 and IgA2), IgM, IgD, and IgE. Among them, IgG is preferable, and IgG2 is more preferable.

The light chain of the antibody of the present invention may be either a $\kappa$ chain or a $\lambda$ chain, and is preferably a $\kappa$ chain.

The most preferred isotype of the antibody of the present invention includes IgG2 whose light chain is a $\kappa$ chain.

<Form>

The form of the antibody of the present invention may be a polyclonal antibody or a monoclonal antibody, but is preferably a monoclonal antibody. The method for producing the monoclonal antibody is not particularly limited, but for example, the monoclonal antibody can be prepared by a hybridoma method as described in "Kohler G, Milstein C., Nature. 1975 Aug 7; 256(5517): 495-497."; a recombinant method as described in U.S. Patent No. 4816567; a method of isolation from a phage antibody library as described in "Clackson et al., Nature. 1991 Aug 15; 352(6336): 624-628." or "Marks et al., J Mol Biol. 1991 Dec 5; 222(3): 581-597.", a method described in "Protein Experiment Handbook, YODOSHA CO., LTD. (2003): 92-96.", and the like.

The antibody of the present invention includes not only a full-length antibody (an antibody having a Fab region and an Fc region) but also a fragment antibody as long as the antibody includes an antigen binding site composed of the CDRs. Examples of such a fragment antibody include Fv antibodies, Fab antibodies, Fab' antibodies, F(ab')$_2$ antibodies, scFv antibodies, dsFv antibodies, diabodies, and nanobodies. The antibody of the present invention also includes a multivalent specific antibody (for example, a bispecific antibody) as long as the antibody includes the antigen binding site. These fragment antibodies and multispecific antibodies can be produced according to conventionally known methods.

The antibody of the present invention may be a conjugated antibody or a conjugated antibody fragment bound to various compounds such as polyethylene glycol, a radioactive substance, and a toxin. In addition, in the antibody of the present invention, the sugar chain bound to the antibody may be altered, or another protein may be fused, as necessary.

2. DNA

The DNA of the present invention is a DNA encoding the "1. Antibodies against pancreatic cancer stem cells". The DNA of the present invention can be obtained, for example, by obtaining at least a region encoding the antibody by PCR or the like using a DNA encoding the antibody as a template. The DNA of the present invention can also be artificially synthesized by a gene synthesis method.

In addition, when a specific mutation is introduced into a specific site of the base sequence of DNA, a mutation-introducing method is known, and for example, a site-directed mutation-introducing method for DNA or the like can be used. As a specific method for converting the base in DNA, for example, a commercially available kit can also be used.

The base sequence of DNA into which a mutation has been introduced can be determined using a DNA sequencer. Once the base sequence is determined, DNA encoding the antibody of the present invention can be obtained by chemical synthesis, PCR using a cloned probe as a template, or hybridization using a DNA fragment having the base sequence as a probe.

In addition, a mutant form of DNA encoding the antibody of the present invention, which has a function equivalent to that before mutation, can be synthesized by a site-directed mutagenesis method or the like. In order to introduce a mutation into a DNA encoding the antibody of the present invention, a known technique such as a Kunkel method, a Gapped duplex method, or a megaprimer PCR method can be used.

The base sequence of the DNA of the present invention can be appropriately designed by those skilled in the art according to the sequence of the CDRs of the antibody of the present invention.

Specifically, the heavy chain CDRs of antibody I can be designed according to the sequences of the heavy chain CDRs of (1-1) to (I-3) or (I-1') to (I-3') above based on SEQ ID NO: 9 that is the base sequence of DNA encoding SEQ ID NO: 1 (for example, the DNA encoding the amino acid sequence from position 26 to position 35 of SEQ ID NO: 1 is composed of the base sequence from position 76 to position 105 of SEQ ID NO: 9) or based on SEQ ID NO: 11 that is the base sequence of DNA encoding SEQ ID NO: 3 (for example, the DNA encoding the amino acid sequence from position 26 to position 35 of SEQ ID NO: 3 is composed of the base sequence from position 76 to position 105 of SEQ ID NO: 11). The light chain CDRs of antibody I can be designed according to the sequences of the light chain CDRs of (I-4) to (I-6) or (I-4') to (I-6') above based on SEQ ID NO: 10 that is the base sequence of DNA encoding SEQ ID NO: 2 (for example, the DNA encoding the amino acid sequence from position 24 to position 34 of SEQ ID NO: 2 is composed of the base sequence from position 70 to position 102 of SEQ ID NO: 10) or based on SEQ ID NO: 12 that is the base sequence of DNA encoding SEQ ID NO: 4 (for example, the DNA encoding the amino acid sequence from position 24 to position 34 of SEQ ID NO: 4 is composed of the base sequence from position 70 to position 102 of SEQ ID NO: 12).

The heavy chain CDRs of antibody II can be designed according to the sequences of the heavy chain CDRs of (II-1) to (II-3) above based on SEQ ID NO: 13 that is the base sequence of DNA encoding SEQ ID NO: 5 (for example, the DNA encoding the amino acid sequence from position 26 to position 35 of SEQ ID NO: 5 is composed of the base sequence from position 76 to position 105 of SEQ ID NO: 13). The light chain CDRs of antibody II can be designed according to the sequences of the light chain CDRs of (II-4) to (II-6) above based on SEQ ID NO: 14 that is the base sequence of DNA encoding SEQ ID NO: 6 (for example, the DNA encoding the amino acid sequence from position 24 to position 34 of SEQ ID NO: 6 is composed of the base sequence from position 70 to position 102 of SEQ ID NO: 14).

The heavy chain CDRs of antibody III can be designed according to the sequences of the heavy chain CDRs of (III-I) to (III-3) above based on SEQ ID NO: 15 that is the base sequence of DNA encoding SEQ ID NO: 7 (for example, the DNA encoding the amino acid sequence from position 26 to position 35 of SEQ ID NO: 7 is composed of the base sequence from position 76 to position 105 of SEQ ID NO: 15). The light chain CDRs of antibody III can be designed according to the sequences of the light chain CDRs of (III-4) to (III-6) above based on SEQ ID NO: 16 that is the base sequence of DNA encoding SEQ ID NO: 8 (for example, the DNA encoding the amino acid sequence from position 24 to position 34 of SEQ ID NO: 8 is composed of the base sequence from position 70 to position 103 of SEQ ID NO: 16).

The base sequence encoding the variable region contained in the DNA of the present invention is not particularly limited as long as a base sequence encoding the CDRs is contained, and the base sequence encoding the framework region is not particularly limited as long as the framework region does not substantially affect the binding activity to pancreatic cancer stem cells. Specific examples of the base sequence encoding the variable region contained in the DNA of the present invention include the following.

Examples of the base sequence encoding the heavy chain variable region of antibody I include SEQ ID NO: 9, and examples of the base sequence encoding the light chain variable region thereof include SEQ ID NO: 10. Examples of the base sequence encoding the heavy chain variable region of antibody II include SEQ ID NO: 13, and examples of the base sequence encoding the light chain variable region thereof include SEQ ID NO: 14. Examples of the base sequence encoding the heavy chain variable region of antibody III include SEQ ID NO: 15, and examples of the base sequence encoding the heavy chain variable region of antibody III include SEQ ID NO: 15, and examples of the base sequence encoding the light chain variable region thereof include SEQ ID NO: 16.

Another example of the base sequence encoding the variable region of each of antibodies I to III includes a base sequence that encodes the variable region containing the CDRs and that has 85% or more sequence identity to the base sequences set forth in SEQ ID NOs: 9, 10, and 13 to 16. The sequence identity is preferably 87% or more, more preferably 92% or more, 95% or more, 96% or more, 97% or more, or 98% or more, particularly preferably 99% or more. The "sequence identity" is as described above.

Still another example of the base sequence encoding the variable region of each of antibodies I to III includes a base sequence of DNA that encodes the heavy chain variable region containing the CDRs and that hybridizes under stringent conditions with DNA containing a base sequence complementary to DNA composed of the base sequences set forth in SEQ ID NOs: 9, 10, and 13 to 16. The "stringent conditions" are as described above.

More specific examples corresponding to the other example or the still other example of the base sequences encoding the variable region of antibody I include SEQ ID NO: 11 that is a more specific example of the base sequence encoding the heavy chain variable region and SEQ ID NO: 12 that is a more specific example of the base sequence encoding the light chain variable region.

The DNA of the present invention is preferably one in which the codon usage is optimized for a host. For example, when E. coli is used as a host, a DNA in which the codon usage is optimized for E. coli is suitable.

3. Recombinant vector

The recombinant vector of the present invention includes the DNA of the present invention described in the "2. DNA". The recombinant vector of the present invention can be obtained by inserting the DNA of the present invention into an expression vector.

As the expression vector, those constructed for genetic recombination from phage, plasmid, or virus capable of autonomously growing in a host are suitable. Specific examples include plasmids derived from E. coli (for example, pET-Blue), plasmids derived from Bacillus subtilis (for example, pUB110), plasmids derived from yeast (for example, pSH19), animal cell expression plasmids (for example, pA1-11, pcDNA 3.1-V5/His-TOPO), bacteriophages such as lambda phage, and virus-derived vectors.

Recombinant vectors contain a regulator operably linked to the DNA of the invention. Examples of the regulator include a promoter, a signal sequence, an origin of replication, a marker gene, an enhancer, a CCAAT box, a TATA box, an SPI site, and a transcription termination sequence, and one or more of these can be used. In addition, operably linked means that various regulators for regulating the DNA of the present invention and the DNA of the present invention are linked in a state where they can operate in a host cell.

The signal sequence is not particularly limited, but examples thereof include a sequence encoding the signal region described above. For example, examples of the heavy chain signal sequence include base sequences set forth in SEQ ID NO: 18 or 22 (encoding SEQ ID NO: 17 or 21, respectively), SEQ ID NO: 26 (encoding SEQ ID NO: 25), and SEQ ID NO: 30 (encoding SEQ ID NO: 29), and examples of the light chain signal sequence include base sequences set forth in SEQ ID NO: 20 or 24 (encoding SEQ ID NO: 19 or 23, respectively) and SEQ ID NO: 28 (encoding SEQ ID NO: 27).

Regarding the signal sequence in the recombinant vector for antibody I, examples of the heavy chain signal sequence include a base sequence set forth in SEQ ID NO: 18 or 22, and examples of the light chain signal sequence include a base sequence set forth in SEQ ID NO: 20 or 24, preferably an amino acid sequence set forth in SEQ ID NO: 20 from the viewpoint of improving antibody production efficiency. Regarding the signal sequence in the recombinant vector for antibody II, examples of the heavy chain signal sequence include the base sequence set forth in SEQ ID NO: 26, and examples of the light chain signal sequence include a base sequence set forth in SEQ ID NO: 28. Regarding the signal sequence contained in the recombinant vector for antibody III, examples of the heavy chain signal sequence include a base sequence set forth in SEQ ID NO: 30, and examples of the light chain signal sequence include an amino acid sequence set forth in SEQ ID NO: 20 from the viewpoint of improving antibody production efficiency.

The recombinant vector for a chimeric antibody can be produced, for example, in the case of a recombinant vector for a human chimerized antibody, by substituting the constant region of a rat antibody having a variable region having amino acid sequences of the respective CDRs with the constant region of a human antibody. Similarly, the recombinant vector for a chimeric antibody can be produced, in the case of a recombinant vector for a mouse chimerized antibody, by substituting the constant region of a rat antibody having a variable region having amino acid sequences of the respective CDRs with the constant region of a mouse antibody. As the constant region of a human antibody, a known constant region can be used.

Specifically, a recombinant vector for the human chimerized antibody can be produced by the following method. First, a DNA encoding a rat heavy chain variable region containing CDRs each having a predetermined amino acid sequence is produced by chemical synthesis, biochemical cleavage, recombination, or the like. A heavy chain expression vector is produced by ligating the resulting DNA encoding the heavy chain variable region with a DNA encoding the human heavy chain constant region, followed by incorporation into an expression vector. A light chain expression vector is produced in the same manner.

In addition, amino acids in the framework region in the variable region of the antibody may be substituted so that the CDRs of the rat-human chimeric antibody form an appropriate antigen binding site (Sato, K. et al., Cancer Research, Vol. 53, p. 851-856 (1993)). A recombinant vector for a mouse chimerized antibody can also be produced in the same manner by using a DNA encoding a mouse constant region instead of a DNA encoding a human constant region.

In addition, a recombinant vector for the humanized antibody can be produced, for example, by transplanting CDRs containing the respective amino acid sequences described above on the framework region of a human antibody (for example, Jones et al., Nature, Vol. 321, p. 522-525 (1986); Riechmann et al., Nature, Vol. 332, p. 323-327 (1988); Verhoeyen et al., Science, Vol. 239, p. 1534-1536 (1988)).

Specifically, a recombinant vector for the humanized antibody can be produced by the following method. A DNA encoding a heavy chain variable region in which CDRs each having a predetermined amino acid sequence are linked to framework regions derived from 4 human antibodies in a predetermined order is produced by chemical synthesis, biochemical cleavage, recombination, or the like. Here, mutations such as substitution, deletion, and/or addition of an amino acid in the framework region may be added so that the respective CDRs of the humanized antibody can form an appropriate antigen binding site (Sato, K. et al., Cancer Research, Vol. 53, p. 851-856 (1993)). A heavy chain expression vector is produced by ligating the resulting DNA encoding the heavy chain variable region with a DNA encoding the human heavy chain constant region, followed by incorporation into an expression vector. Similarly, a light chain-heavy chain expression vector is produced. As the constant region of a human antibody, a known constant region can be used.

4. Transformant

The transformant is obtained by transforming a host using the DNA of the present invention or the recombinant vector.

The host for use in production of the transformant is not particularly limited as long as the host can introduce a gene, can autonomously grow, and can express the trait of the gene of the present invention, but examples thereof include cells of humans and mammals excluding humans (for example, rat, mouse, guinea pig, rabbit, cow, monkey etc.), more specifically, Chinese hamster ovary cells (CHO cells), monkey cells COS-7, human embryonic kidney cells (for example, HEK 293 cells (Expi293F, 293-F, HEK293, 293T, etc.)), mouse myeloma cells (for example, Sp2/0 or NS0); insect cells; plant cells; Bacteria belonging to genus Escherichia such as Escherichia coli, genus Bacillus such as Bacillus subtilis, genus Pseudomonas such as Pseudomonas putida, etc.; actinomycetes, etc.; yeast etc.; and filamentous fungi etc.

The transformant can be obtained by introducing the DNA of the present invention or the recombinant vector into a host. The method for introducing the DNA of the present invention or the recombinant vector is not particularly limited as long as the gene of interest can be introduced into a host. In addition, the site into which the DNA is introduced is not particularly limited as long as the gene of interest can be expressed, but may be on a plasmid or on a genome. Specific examples of the method for introducing the DNA of the present invention or the recombinant vector include a recombinant vector method and a genome editing method.

Conditions for introducing the DNA of the present invention or the recombinant vector into a host may be appropriately set according to the introduction method, the type of host, and the like. When the host is bacteria, examples of the method include a method using a competent cell with calcium ion treatment and an electroporation method. When the host is a yeast, examples of the method include an electroporation method, a spheroplast method, and a lithium acetate method. When the host is an animal cell, examples of the method include an electroporation method, a calcium phosphate method, and a lipofection method. When the host is an insect cell, examples of the method include a calcium phosphate method, a lipofection method, and an electroporation method. When the host is a plant, examples of the method include an electroporation method, an Agrobacterium method, a particle gun method, and a PEG method.

5. Method for producing antibody

The antibody of the present invention described in the "1. Antibodies against pancreatic cancer stem cells" can be produced by culturing the transformant described in the "4. Transformant". The culture conditions of the transformant may be appropriately set taking into consideration of the nutritional physiological properties of a host, but liquid culture is preferable. From the obtained culture, the antibody of the present invention of interest can be collected and purified.

6. Pharmaceutical composition

The present invention provides a pharmaceutical composition containing the antibody of the present invention described in the "1. Antibodies against pancreatic cancer stem cells". The pharmaceutical composition of the present invention can exhibit a pharmacological effect based on the binding ability to pancreatic cancer stem cells using the antibody of the present invention as an active ingredient.

The pharmaceutical composition of the present invention only needs to contain an effective amount of the antibody of the present invention, and may further contain a pharmaceutically acceptable carrier or additive. Examples of such a carrier or additive include a surfactant, an excipient, a colorant, an aromatizing agent, a preservative, a stabilizer, a buffer, a pH buffer, a disintegrant, a solubilizing agent, a solubilizing aid, an isotonizing agent, a binder, a disintegrant, a lubricant, a diluent, and a flavoring agent.

In addition, the administration form of the pharmaceutical composition of the present invention may be either oral or parenteral, and specific examples thereof include oral administration; and parenteral administration such as intravenous administration, intramuscular administration, intraperitoneal administration, subcutaneous administration, nasal administration, pulmonary administration, transdermal administration, transmucosal administration, and intraocular administration.

Regarding the formulation form of the pharmaceutical composition of the present invention, the formulation form can be appropriately set according to the employed administration form. For example, when used in oral administration, the pharmaceutical composition of the present invention may be prepared in a formulation form such as a powder, a granule, a capsule, a syrup, or a suspension, and when used in parenteral administration, the pharmaceutical composition of the present invention may be prepared in a formulation form such as a liquid, a suspension, an emulsion, a spray, a suppository, or an eye drop.

The pharmaceutical composition of the present invention can be used for treatment of pancreatic cancer by utilizing the antitumor effect of the antibody of the present invention.

The dose and the number of times of administration of the pharmaceutical composition of the present invention vary depending on the administration form, the age and body weight of a patient, the type of disease and the degree of symptom, and the like, and cannot be uniformly defined. For example, in the case of human, an amount corresponding to 120 mg to 480 mg in terms of the weight of the antibody of the present invention may be administered at a frequency of about once every 7 to 30 days per administration. In order to reduce the number of times of administration, a sustainedrelease preparation can be used, or administration can be carried out in small amounts over a long period of time with an osmotic pump or the like.

7. Extracorporeal diagnostic agent

The present invention provides an extracorporeal diagnostic agent composed of the antibody of the present invention described in the "1. Antibodies against pancreatic cancer stem cells". The extracorporeal diagnostic agent of the present invention can exhibit a diagnostic effect based on the binding ability to pancreatic cancer stem cells using the antibody of the present invention as a tumor marker.

Since the antibody of the present invention has a binding property to pancreatic cancer stem cells for any of antibody I, antibody II and antibody III, the extracorporeal diagnostic agent of the present invention can be used for diagnosis of pancreatic cancer. Among the antibodies of the present invention, since antibody I and antibody II specifically bind to pancreatic cancer stem cells, the extracorporeal diagnostic agent of the present invention is more preferably composed of antibody I and antibody II. On the other hand, since antibody III is an anti-EpCAM antibody and has binding properties to not only pancreatic cancer stem cells but also cancer cells having EpCAM, when composed of antibody III, the extracorporeal diagnostic agent of the present invention can be used for diagnosis of cancer expressing EpCAM. The cancer expressing EpCAM is not particularly limited, but examples thereof include renal cell cancer, hepatocellular cancer, urothelial cancer, breast cancer, squamous cell cancer, and lung cancer.

Examples of the detection target of the extracorporeal diagnostic agent of the present invention include cancer cells such as pancreatic cancer stem cells collected from a living body to be diagnosed, or extracellular vesicles (such as exosomes) derived from cancer cells considered to reflect the surface state of the cancer cells. When used as a detection target, the extracellular vesicle derived from a cancer cell is preferable from the viewpoint that a non-invasive diagnosis can be expected without performing biopsy, and an improved diagnostic sensitivity can be expected in pancreatic cancer diagnosis. Specific examples of the biological sample including the detection target include a biopsy sample, a blood collection sample, and a urine collection sample.

The method for analyzing a biological sample for diagnosis of cancer using the extracorporeal diagnostic agent of the present invention includes a step of bringing the extracorporeal diagnostic agent of the present invention (that is, the antibody of the present invention) into contact with a biological sample to immunomeasure an antigenantibody reaction.

The immunoassay may be based on any method such as a sandwich method, a competition method, or an agglutination method, but is preferably based on a sandwich method. In addition, examples of the immunoassay include an enzyme-linked immuno-sorbent assay (ELISA), fluorescence immunoassay, and radioisotope immunoassay, depending on the type of label, but from the viewpoint of simplicity and/or rapidity of measurement, an enzyme-linked immuno-sorbent assay is preferable.

Examples of specific aspects of the extracorporeal diagnostic agent of the present invention include an aspect immobilized on a substrate. That is, the present invention also provides an antibody substrate containing the above-described extracorporeal diagnostic agent and a substrate on which the extracorporeal diagnostic agent is immobilized. The material of the substrate is not particularly limited as long as it is in a solid phase, but examples thereof include polystyrene, polyethylene, polypropylene, polyester, polyacrylonitrile, polyvinyl chloride, fluororesin, crosslinked dextran, paper, silicon, glass, metal, and agarose, and examples of the form thereof include a plate and a film. As a method for immobilizing the extracorporeal diagnostic agent of the present invention (that is, the antibody of the present invention) on a substrate, a method known in the technical field of producing antibody substrates is used without particular limitation. In the use of the antibody substrate, immunoassay based on a sandwich method can be performed using the extracorporeal diagnostic agent of the present invention as a capture antibody.

Another example of specific aspects of the extracorporeal diagnostic agent of the present invention includes an aspect contained in an extracorporeal diagnostic composition. That is, the present invention also provides an extracorporeal diagnostic composition containing the extracorporeal diagnostic agent. The extracorporeal diagnostic agent contained in the extracorporeal diagnostic agent composition may be in the form of the antibody itself or in the form in which the antibody is immobilized on insoluble particles. In addition to the extracorporeal diagnostic agent, the extracorporeal diagnostic composition can contain one or more components selected from salts, sugars, polyhydric alcohols, surfactants, proteins (other than the extracorporeal diagnostic agent), stabilizers, preservatives, buffers, and the like.

The antibody substrate and the extracorporeal diagnostic composition may be provided in the form of a kit containing another reagent and/or an instrument according to the measurement principle of immunoassay and the type of label. For example, when the enzyme-linked immuno-sorbent assay is selected, the kit can further contain one or more items selected from a reaction vessel or a reaction plate, a chromogenic substrate solution, a reaction stop solution, a washing solution, a standard solution, and the like, in addition to the antibody substrate and the extracorporeal diagnostic composition.

EXAMPLES

Hereinafter, a more detailed description is made of the present invention with reference to Examples, but the present invention is not to be construed as being limited to these Examples.

Test Example 1: Production of monoclonal antibody against human pancreatic cancer stem cell line KMC07

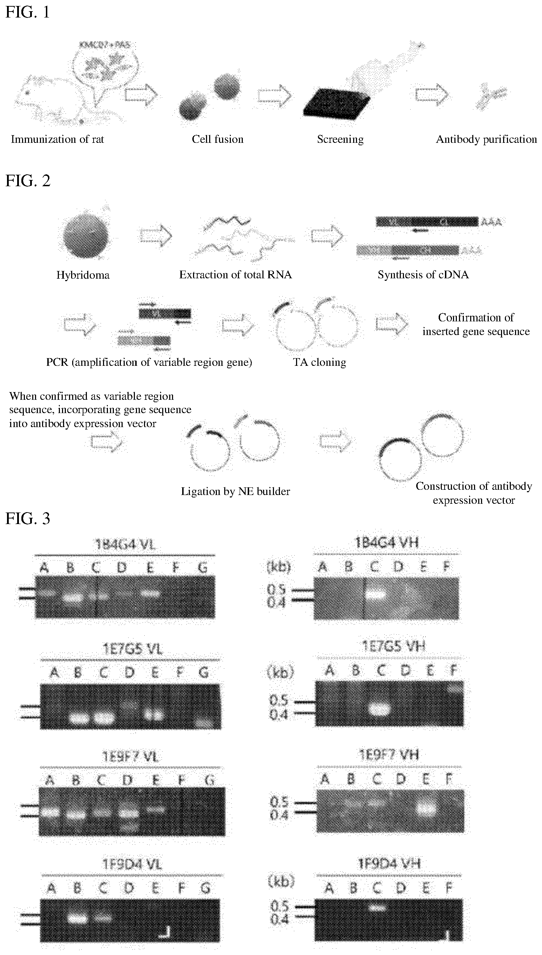

The flow of antibody production performed in this Test Example is shown in Fig. 1.

1-1. Materials and methods

Cell culture

For BxPC-3 (human pancreatic adenocarcinoma cell lines) and MCF-7 (human breast cancer cell lines) was used 10% FBS [SIGMA, F7524]-containing D-MEM [Wako, 044-29765], for KP-3 (human pancreatic adenosquamous carcinoma cell lines), HARA (human lung cancer cell lines), HARA-B2 (human lung cancer cell bone metastasis lines), PC-3 (human prostate cancer cell lines), and HeLa (human cervical carcinoma cell lines) was used 10% FBS-containing RPMI-1640 [Wako, 189-02025], for NHDF (human fibroblast cell lines) was used 20% FBS-containing D-MEM, and for Sp2 (mouse myeloma cell lines) was used 10% FBS-containing Hybridoma-SFM [Gibco, 12300-067], followed by culture at 37°C under 5% by volume $CO_2$.

Culture of KMC07

KMC07 is a human pancreatic tumor progenitor cell line (human pancreatic cancer stem cell) established by the present inventor from pancreatic cancer tissues isolated from a human pancreatic cancer patient (age: 69 years old, sex: male, pathological subtype: tubular adenocarcinoma, metastasis: liver/peritoneum/intraperitoneal lymph node, origin: peritoneal exudate) treated with an anticancer agent by administration of gemcitabine (Shimizu K., Nishiyama T., Hori Y. (2017) Gemcitabine Enhances Kras-MEK-Induced Matrix Metalloproteinase-10 Expression Via Histone Acetylation in Gemcitabine-Resistant Pancreatic Tumor-initiating Cells. Pancreas. 46, 268-275). KMC07 was co-cultured with PA6 (mouse fibroblast cell lines). PA6 was cultured using 10% FBS-containing MEMα [Wako, 137-17215] at 37°C under 5% CO2 until it became confluent. KMC07 was seeded on PA6 rinsed with PBS, followed by culture using KMC Medium (Stem Medium [KAC, KSDSRK100], 25 U/ml penicillin [Gibco, 15070-063], 25 μg/ml streptomycin [Gibco 15070-063], 0.1 μM 2-mercaptoethanol [Wako, 137-07521]) at 37°C under 5% by volume

$CO_2$.

## Production of hybridoma

In order to produce antibodies against KMC07, one WKY/Izm rat [SLC, 8-weeks old female] was immunized twice using KMC07 as an antigen. In the initial immunization, a mixed PBS suspension of about $1.0 \times 10^7$ cells of KMC07 and PA6 was mixed with Titer Max Gold [CYT, G-1X5] as an adjuvant at a volume ratio of 1 : 1, followed by sufficient suspension using a sonicator. Into the respective foot soles of the WKY/Izm rat, 100 μl was subcutaneously injected. The booster was performed 15 days after the immunization. In the booster, only a mixed PBS suspension of about 6.7 $\times 10^5$ cells of KMC07 and PA6 was subcutaneously injected into the respective left and right buttocks in an amount of 100 μl. Four days after the booster, lymphocytes isolated from the iliac lymph node and Sp2 were mixed at a ratio of 3 : 1, and cells (hybridomas) fused by a PEG method were seeded on a 96 well plate. Selection was performed using HAT medium (Hybridoma-SFM, 10% FBS, 100 mM hypoxanthine [SIGMA, H9377-1G], 0.4 mM aminopterin [SIGMA, A3411-10MG], 16 mM thymidine [Wako, 205-08091], 1 ng/ml mouse IL-6, 100 U/ml penicillin [Wako, 161-23181], 100 μg/ml streptomycin [Wako, 161-23181], 0.25 μg/ml amphotericin B [Wako, 161-23181]) as a medium.

## Immunostaining

When using KMC07: PA6 was first seeded on a 1.5 μg/ml poly-L-ornithine [SIGMA, P3655 -50MG]-coated 96 well immunostaining plate [Thermo Scientific, 164588]. PA6 was cultured in 10% FBS-containing MEMα for 2 days. After 2 days, the culture supernatant of PA6 was removed, followed by rinsing with PBS. Subsequently, KMC07 and PA6 were suspended in KMC Medium so as to be about 1,500 cells/well in combination, followed by seeding on each well. After a further 3 days, the culture supernatant was removed, followed by addition of the hybridoma culture supernatant diluted 10 times as a primary antibody, and incubation for 1 hour. After 1 hour, cells were fixed by treatment with a 3.7% solution of formaldehyde [Wako, 064-00406] in PBS for 15 minutes. Finally, a solution of a secondary antibody (Alexa 488 anti-rat IgG (H+L) [Invitrogen™, A11006]) in PBS containing Hoechst (registered trademark) 33342 [Invitrogen™, H3570] was added, followed by incubation for 30 minutes. Observation was performed with a fluorescence microscope (OLYMPUS, IX71).

When using other cells: Two days before immunostaining, cells were seeded on an 8-well immunostaining plate [MP Biomedicals, 6040805E]. The hybridoma culture supernatant was directly used as a primary antibody, followed by incubation for 1 hour. After 1 hour, cells were fixed by treatment with a 3.7% solution of formaldehyde in PBS for 15 minutes. Furthermore, incubation was performed with a solution of a secondary antibody (Alexa 488 anti-rat IgG (H+L)) in PBS containing Hoechst (registered trademark) 33342 for 30 minutes. Finally, a discoloration inhibitor DABCO [SIGMA, D-2522] was added, followed by observation with a fluorescence microscope.

## Limiting dilution

Cloning of the hybridoma was performed by seeding on a 96 well plate the hybridoma diluted so as to be 1 cell/well using a HAT-free medium (Hybridoma-SFM, 10% FBS, 1 ng/ml mouse IL-6, 100 U/ml penicillin, 100 μg/ml streptomycin, 0.25 μg/ml amphotericin B). After several days, it was confirmed by a microscope that single colonies were formed.

## Antibody purification

Hybridomas were cultured in a bovine serum-free medium (Hybridoma-SFM, 1 ng/ml mouse IL-6), and from the culture supernatant, antibodies were purified. For the purification, an ammonium sulfate precipitation method was used, and a solution of the purified antibodies was finally substituted with PBS. The concentration of the purified antibodies was determined using Pierce™ BCA Protein Assay Kit [Thermo Scientific, 23227]. In addition, isotyping of the respective monoclonal antibodies was performed using Rat Immunoglobulin Isotyping ELISA Kit [BD Biosciences, 557081].

## 1-2. Result

## 1-2-1. Production of monoclonal antibody against human pancreatic cancer stem cell line KMC07

Monoclonal antibodies specifically recognizing the human pancreatic cancer stem cell line KMC07 was produced using a rat iliac lymph node method. In this study, it is an object to produce an antibody targeting the cell membrane of KMC07. Here, KMC07 was mixed with PA6 for immunization of rats. For the first immunization, a mixed solution of a cell suspension and an adjuvant was used. After 15 days from the first immunization, only the cell suspension was used for booster. Four days after the booster, $6.0 \times 10^8$ lymphocytes were isolated from the iliac lymph node, and $1.5 \times 10^8$ lymphocytes among them were subjected to cell fusion with Sp2 by a PEG method. The produced hybridomas were seeded on 10 96 well plates. The hybridoma culture supernatant was screened by immunostaining. As a result of immunostaining for KMC07, about 40% of the wells were positive for KMC07. Among them, hybridomas in wells with particularly strong signals were selected for cloning using a limiting dilution method. Thereafter, immunostaining of KMC07 and PA6 was performed using the hybridoma culture supernatant forming a single colony. As a result, 5 clones of KMC07-positive/PA6-negative antibody (1B4G4: corresponding to antibody I (IgG2b, κ), 1E2D3 (IgG2a, λ), 1E7G5: corresponding to antibody I (IgG2b, κ), 1E9F7: corresponding to antibody II (IgG2a, κ), 1F9D4: corresponding to antibody III (IgG2b, κ)) were successfully established. In addition, it was confirmed that each antibody did not recognize normal human cells by performing immunostaining using NHDF (human fibroblast cell lines).

1-2-2. Reactivity against various human cancer cell line

In order to examine the binding property and specificity of the purified antibodies 1B4G4, 1E2D3, 1E7G5, 1E9F7, and 1F9D4 to KMC07, immunostaining was performed using various human-derived cancer cell lines. As the human cancer cell lines, BxPC-3 (pancreatic adenocarcinoma cell lines), KP-3 (pancreatic adenosquamous carcinoma cell lines), HARA (lung cancer cell lines), HARA-B2 (lung cancer cell bone metastasis lines), MCF-7 (breast cancer cell lines), HeLa (cervical carcinoma cell lines), and PC-3 (prostate cancer cell lines) were used. The intensity of a stain signal was evaluated in the following five stages. Incidentally, KP-3 is a pancreatic cancer cell line that has been established, but since it has recently become clear that a small number of cancer stem cell-like cells are also contained in the established cancer cell line (Christine M F. & Charlotte K. (2008). Human breast cancer cell lines contain stem-like cells that self-renew, give rise to phenotypically diverse progeny and survive chemotherapy. Breast Cancer Research. 10, R 25.), it is considered that pancreatic cancer stem cell-like cells are also present in KP-3. The results are shown in Table 1.

-: No stain signal was obtained
±: A slight stain signal was obtained
+: A stain signal was obtained
++: A strong stain signal was obtained
+++: A stronger stain signal was obtained

[Table 1]

| Human Cell Lines | Antibody I | | Antibody I | Antibody II | Antibody III |
|---|---|---|---|---|---|
| | 1B4G4 | 1E2D3 | 1E7G5 | 1E9F7 | 1F9D4 |
| KMC07 (Human Pancreatic Cancer Stem Cell Lines) | +++ | +++ | +++ | +++ | +++ |
| KP-3 (Pancreatic Adenosquamous Carcinoma Cell Lines) † | + | +++ | + | - | +++ |
| BxPC-3 (Pancreatic Adenocarcinoma Cell Lines) | - | +++ | - | - | +++ |
| HARA (Lung Cancer Cell Lines) | - | ++ | - | - | +++ |
| HARA-B2 (Lung Cancer Cell Bone Metastasis Lines) | - | +++ | - | - | +++ |
| MCF-7 (Breast Cancer Cell Lines) | - | +++ | - | - | +++ |
| HeLa (Cervical Carcinoma Cell Lines) | - | - | - | - | - |
| PC-3 (Prostate Cancer Cell Lines) | ± | ± | ± | ± | ± |
| † There is a report that pancreatic cancer stem cells are contained | | | | | |

As shown in Table 1, all antibodies exhibited a binding property to pancreatic cancer stem cells (exhibited positive).
In addition, 1B4G4 (antibody I), 1E7G5 (antibody I), and 1E9F7 (antibody II) exhibited a specific binding property to pancreatic cancer stem cells. Among them, as to the point that 1B4G4 (antibody I) and 1E7G5 (antibody I) exhibited positive for KP-3, it is considered that pancreatic cancer stem cell-like cells in KP-3 were specifically recognized. In addition, these antibodies were also tentatively positive for PC-3, but since the stain signal intensity was extremely weaker than that for KMC07, they can be evaluated as specific to pancreatic cancer stem cells.

On the other hand, since 1E2D3 and 1F9D4 (antibody III) were positive for all cells except HeLa, it is considered that they exhibit binding properties to general cancer cells including pancreatic cancer stem cells.

All antibodies were negative for HeLa. It is considered that HeLa is repeatedly passaged so that the character is changed, and various proteins are expressed. Accordingly, a stain signal is often obtained for HeLa when an antibody that recognizes cancer cells is used. However, since the antibodies established in the Test Example were negative for HeLa, it was suggested that the antibodies established in the Test Example recognized a protein highly expressed in non-established cancer cells.

Test Example 2: Gene cloning of antibody against KMC07 and production of recombinant chimeric antibody

Gene cloning and production of recombinant chimeric antibodies were performed for 1B4G4 (antibody I), 1E7G5 (antibody I), 1E9F7 (antibody II), and 1F9D4 (antibody III). A rough flow of the antibody gene cloning performed in the Test Example is shown in Fig. 2. The list of the used primers is shown in Tables 2A to 2B.

2-1. Materials and methods

Cell culture

For HEK 293 T (human embryonic kidney cell lines) was used 10% FBS-containing D-MEM, followed by culture at 37°C under 5% by volume $CO_2$. For Expi293F (suspension system 293 cell lines) was used 2 mmol/l L-alanyl-L-glutamine solution [Wako, 016-21841] HE200-containing [gmep (registered trademark), HE200-0010], followed by shaking culture at 37°C under 5% by volume $CO_2$ at 125 rpm. The method for culturing KMC07 was according to "Culture of KMC07" in "1-1. Materials and methods" of Test Example 1.

RNA extraction from hybridoma and synthesis of antibody variable region cDNA

Total RNA was extracted from half the amount of hybridomas cultured in a P10 dish until it became confluent. First, the hybridomas were centrifuged at 1,800 rpm for 5 minutes. To the pellet, 1 ml of TRIzol [ambion, 15596-026] was added, and the pellet was allowed to stand at room temperature for 5 minutes. Then, 200 $\mu$l of chloroform [SIGMA, 319988-500ML] was added, and the mixture was allowed to stand at room temperature for 2 to 3 minutes. The mixture was centrifuged at 12,000 g for 15 minutes at 4°C so that the mixture separated into 2 layers. The upper layer was collected, and 500 $\mu$l of isopropanol [Wako, 166-04836] was added to and mixed with the collected upper layer. Then, the mixture was allowed to stand at room temperature for 10 minutes. After centrifugation at 12,000 g for 10 min at 4°C, the supernatant was removed, followed by addition of 1 ml of 75% ethanol solution. After centrifugation at 7,500 g for 5 min at 4°C, the supernatant was removed. After air-drying, dissolution was performed in 20 $\mu$l of RNase free water [TaKaRa, 9012]. Based on the extracted RNA, cDNA of each of the heavy chain and the light chain was synthesized using SuperScript (trademark) III Reverse Transcriptase [Invitrogen, 18080-093]. Reverse transcription PCR solution 1 (1 $\mu$g of RNA, 4 $\mu$l of 2.5 mM dNTPs, and 0.4 $\mu$l of 5 $\mu$M Primer, diluted to 13 $\mu$l with autoclaved, distilled water) was reacted at 65°C for 5 minutes, followed by cooling to 25°C. A reverse transcription PCR solution 2 (4 $\mu$l of 5× First-Strand Buffer, 1 $\mu$l 0.1 M DTT, 1 $\mu$l of 40 U/$\mu$l Recombinant RNasin (registered trademark) Ribonuclease Inhibitor [Promega, N251A], 1 $\mu$l of 200 U/$\mu$l SuperScript (trademark) IIIRT) was added, followed by reaction at 55°C for 30 minutes at 70°C for 15 minutes. At this time, primer 1 was used for the heavy chain, and primer 2 was used for the light chain.

Production of antibody gene expression vector

Using cDNA as a template, PCR was performed using primer sets 4 to 9 and primer 1 for the heavy chain, and primer sets 10 to 16 and primer 2 for the clone of the light chain κ to amplify the heavy chain variable region (VH) and the light chain variable region (VL), respectively. The obtained PCR product was inserted into pGEM (registered trademark)-T-easy vector [Promega, A1360]. At this time, the colonies selected by the blue-white selection were subjected to PCR using primer 17 and primer 18 (in the case of light chain, 3 types with addition of primer 19), which confirmed that the hybridoma-derived gene was correctly inserted. Then, the VH and VL gene fragments of each antibody were amplified using the primers shown in Table 3. The obtained PCR products were inserted into animal expression vectors pCEC2.1-rIgG2a and pCEC2.1-rIgK incorporating heavy chain and light chain constant region genes in advance by NE builder [NEB, E2621S].

Introduction of antibody expression vector into cell

When using HEK293T: To 221 μl of FBS-free D-MEM were added 1.3 μg each of the heavy chain and light chain expression vectors. To this, 4 μl of 1 mg/ml PEI [Polysciences, 23966] was added, and the mixture was allowed to stand at room temperature for 20 minutes. Then, 1.3 ml of 2% FBS-containing D-MEM was added. The culture supernatant of HEK293T cultured in a 12 well plate was removed, and a prepared solution was added. Incubation was performed at 37°C under 5% by volume $CO_2$ for 4 days, and the supernatant was collected.

When using Expi 293 F: To 1.5 ml of Opti-MEM [Gibco, 31985-062], 120 μl of 1 mg/ml PEI MAX [Polysciences, 24765] was added, and the mixture was allowed to stand at room temperature for 5 minutes. Separately, 15 μg each of the heavy chain and light chain expression vectors were added to 1.5 ml of Opti-MEM to prepare a plasmid solution. The prepared solution was added to and mixed with a PEI MAX solution, and the mixture was allowed to stand at room temperature for 20 minutes. The prepared solution was added to $7.5 \times 10^7$ cells of Expi293F suspended in 27 ml of Expi293 Expression Medium [Gibco, A14351-01], followed by shaking culture at 37°C under 5% by volume $CO_2$ at 125 rpm. After 20 h, 75 μl of 0.5 M sodium valproate [TCI, S0894] and 120 μl of 1 M sodium propionate [Wako, 194-03012] were added. Seven days after transfection, the supernatant was collected.

Immunostaining using KMC07

This was according to "Immunostaining" in "1-1. Materials and methods" of Test Example 1. When using as a primary antibody a culture supernatant of HEK293T into which an anti-KMC07 antibody expression vector had been introduced, the culture supernatant was used without dilution.

Antibody purification using Protein A

The antibody was forcibly expressed in Expi293F, and the antibody was purified from the culture supernatant using Protein A. To 30 ml of the culture supernatant from which the cells had been removed, 1.5 ml of 1 M Tris-HCl and 0.2 ml of a solution of Protein A (rProtein A Sepharose (trademark) FastFlow [GE Healthcare, 17-1279-01]) in PBS were added, followed by shaking at 4°C overnight. The next day, a column was packed with Protein A reacted with the antibody, followed by replacement with a TBS solution. The antibody solution was collected into 5 fractions by applying to the column 300 μl of Gentle Ag/Ab Elution Buffer, pH 6.6 [Thermo Scientific, 21027] at a time. For each fraction, the absorbance at 280 nm was measured, and the solution having the peak was collected in a dialysis tube. By dialysis, the purified antibody solution was finally replaced with PBS. The concentration of the purified antibodies was determined using Pierce (trademark) BCA Protein Assay Kit.

Human chimerization and mouse chimerization of rat heavy chain gene

Only CH2 and CH3 corresponding to the Fc region of the rat antibody were humanized and murinized. A rat heavy chain gene fragment (VH to Hinge) of each antibody was amplified using a rat heavy chain expression vector as a template and primers shown in Table 4. The obtained PCR products were inserted into animal expression vectors pCEC2.1-hIgG1 and pUC19-mIgG2a incorporating human and mouse Fc region genes in advance by NE builder.

Recombination of light chain SP sequence (signal sequence)

Production of 1E7SP-1F9D4 light chain expression vector: The 1E7G5 light chain SP sequence gene was amplified by PCR using a 1E7G5 light chain expression vector as a template, and primer 23 and primer 35, and the 1F9D4 VL gene was amplified by PCR using a 1F9D4 light chain expression vector as a template, and primer 36 and primer 2. Next, PCR was performed using the two obtained PCR products as templates, and primer 23 and primer 2 to connect the respective gene fragments. Finally, the obtained PCR product was inserted into an animal expression vector pCEC 2.1-rIgK incorporating a light chain constant region gene in advance.

Production of 1B4SP-1E7G5, 1B4SP-1F9D4 light chain expression vector: The 1B4G4 light chain SP sequence gene was amplified by PCR using a 1B4G4 light chain expression vector as a template, and primer 21 and primer 37. The 1E7G5 and 1F9D4 VL genes were amplified by PCR using 1E7G5 and 1F9D4 light chain expression vectors as templates, respectively, and primer 38 and primer 2. Next, PCR was performed using the two obtained PCR products (1B4G4 light chain SP gene and each antibody VL gene) as templates, and primer 21 and primer 2 to connect the respective gene fragments. Finally, the obtained PCR product was inserted into an animal expression vector pCEC 2.1-rIgK incorporating a light chain constant region gene in advance.

Dot blot

On a membrane (HybondTM-C Extra [GE Healthcare, BPN303E]) was blotted 0.5 μl of culture supernatant of Expi293F by which each antibody had been forcibly expressed. At this time, a stock solution, and 10 times, 20 times to 640 times serially diluted solutions of the culture supernatant were used. The membrane was air-dried and then immersed in a 3% solution of skim milk in TBS-T for 30 minutes. After blocking, the membrane was incubated for 30 minutes with a secondary antibody (Anti-Rat IgGAP, antibody produced in rabbit) diluted with Can Get Signal Solution II. Finally, color development was performed using a BCIP/NBT solution.

[Table 2A]

| No. | Name of primer | Sequence (5'-3') | Primer length |
|---|---|---|---|
| 1 | RaIgGVH3'-1 | TTCCAGGAGCCAGTGGATAGACAGATG | 27 mer |
| 2 | RaIgkVL3'-1 | AGATAGATACAGTTGGTGCAGCATCAGC | 28 mer |
| 4 | MuIgVH5-A | GGGAATTCATGRASTTSKGGYTMARCTKGRTTT | 33 mer |
| 5 | MuIgVH5-B | GGGAATTCATGRAATGSASCTGGGTYWTYCTCTT | 34 mer |
| 6 | MuIgVH5-C-1 | ACTAGTCGACATGGACTCCAGGCTCAATTTAGTTTTCCT | 39 mer |
|  | MuIgVH5-C-2 | ACTAGTCGACATGGCTGTCYTRGBGCTGYTCYTCTG | 36 mer |
|  | MuIgVH5-C-3 | ACTAGTCGACATGGVTTGGSTGTGGAMCTTGCYATTCCT | 39 mer |
| 7 | MuIgVH5-D-1 | ACTAGTCGACATGAAATGCAGCTGGRTYATSTTCTT | 36 mer |
|  | MuIgVH5-D-2 | ACTAGTCGACATGGRCAGRCTTACWTYYTCATTCCT | 36 mer |
|  | MuIgVH5-D-3 | ACTAGTCGACATGATGGTGTTAAGTCTTCTGTACCT | 36 mer |
| 8 | MuIgVH5-E-1 | ACTAGTCGACATGGGATGGAGCTRTATCATSYTCTT | 36 mer |
|  | MuIgVH5-E-2 | ACTAGTCGACATGAAGWTGTGGBTRAACTGGRT | 33 mer |
|  | MuIgVH5-E-3 | ACTAGTCGACATGGRATGGASCKKDRTCTTTMTCT | 35 mer |
| 9 | MuIgVH5-F-1 | ACTAGTCGACATGAACTTYGGGYTSAGMTTGRTTT | 35 mer |
|  | MuIgVH5-F-2 | ACTAGTCGACATGTACTTGGGACTGAGCTGTGTAT | 35 mer |
|  | MuIgVH5-F-3 | ACTAGTCGACATGAGAGTGCTGATTCTTTTGTG | 33 mer |
|  | MuIgVH5-F-4 | ACTAGTCGACATGGATTTTGGGCTGATTTTTTTTATTG | 38 mer |

[Table 2B]

| No. | Name of primer | Sequence (5'-3') | Primer length |
|---|---|---|---|
| 17 | M13_F | GTAAAACGACGGCCAGT | 17 mer |
| 18 | M13_R | CAGGAAACAGCTATGAC | 17 mer |
| 19 | sp2_abvk-1 | AGTACATCTGGCTATAGTTATATGCAC | 27 mer |
| 20 | 1B4G4_VH_C-3_F | TGTCTCATCATTTTGGCAAAGCCACCATGGCTGTCCTAGTGCTGC | 45 mer |
| 21 | 1B4G4_VL_A-1_F | TGTCTCATCATTTTGGCAAAGCCACCATGGAGTCACATTCTCAGGT | 46 mer |
| 22 | 1E7G5_VH_C-4_F | TGTCTCATCATTTTGGCAAAGCCACCATGGCTGTCTTAGTGCTGTTCC | 48 mer |
| 23 | 1E7G5_VL_G-3_F | TGTCTCATCATTTTGGCAAAGCCACCATGAAGTTGCCTGTTAGGCTGT | 48 mer |
| 24 | 1E9F7_VH_E2-5_F | TGTCTCATCATTTTGGCAAAGCCACCATGGGATGGAGCTGTATCAT | 46 mer |
| 25 | 1E9F7_VL_A-1_F | TGTCTCATCATTTTGGCAAAGCCACCATGGAGTCACAGACCCAGG | 45 mer |

(continued)

| No. | Name of primer | Sequence (5'-3') | Primer length |
|---|---|---|---|
| 26 | 1F9D4_VH_C-1_F | TGTCTCATCATTTTGGCAAAGCCACCATGGACTCCAGGCTCAATTTAGTT | 50 mer |
| 27 | 1F9D4_VL_C-4_F | TGTCTCATCATTTTGGCAAAGCCACCATGGAGTCAGACACACTGCTG | 47 mer |
| 28 | 1B4G4_VH_F1 | ACGGTATCGATAAGCTTGTGGCCACCATGGCTGTCCTAGTGCTGC | 45 mer |
| 29 | 1E7G5_VH_F1 | ACGGTATCGATAAGCTTGTGGCCACCATGGCTGTCTTAGTGCTGT | 45 mer |
| 30 | 1E9F7_VH_F1 | ACGGTATCGATAAGCTTGTGGCCACCATGGGATGGAGCTGTATC | 44 mer |
| 31 | 1F9D4_VH_F1 | ACGGTATCGATAAGCTTGTGGCCACCATGGACTCCAGGCTCA | 42 mer |
| 32 | rIgG_Hinge_R1 | CACCCAAGAGGTTAGGTGCTGTACAGCCGCAGGGGTTGC | 39 mer |
| 33 | CAG_kozak_ATG_F | CTGTCTCATCATTTTGGCAAAGCCACCATG | 30 mer |
| 34 | rIgG_Hinge_R2 | CCGAGCAGTTCTGGAGCTGCTGTACAGCCGCAGGGGTTGC | 40 mer |
| 35 | 1E7G5_SP_R | GGAGACTGGGTCATCTGGATGTC | 23 mer |
| 36 | 1F9D4_VL_F | GACATCCAGATGACCCAGTCTCC | 23 mer |
| 37 | 1B4G4_SP_R | GCTGGGAGGAAGAGCACCAA | 20 mer |
| 38 | 1B4G4_SP_F | TTGGTGCTCTTCCTCCCAGC | 20 mer |

[Table 3]

| | Forward primer | Reverse primer |
|---|---|---|
| 1B4G4 Heavy Chain | No. 20 | No. 1 |
| 1B4G4 Light Chain | No. 21 | No. 2 |
| 1E7G5 Heavy Chain | No. 22 | No. 1 |
| 1E7G5 Light Chain | No. 23 | No. 2 |
| 1E9F7 Heavy Chain | No. 24 | No. 1 |
| 1E9F7 Light Chain | No. 25 | No. 2 |
| 1F9D4 Heavy Chain | No. 26 | No. 1 |
| 1F9D4 Light Chain | No. 27 | No. 2 |

[Table 4]

|  | Forward primer | Reverse primer |
|---|---|---|
| mouse 1B4G4 | No. 28 | |
| mouse 1E7G5 | No. 29 | No. 32 |
| mouse 1E9F7 | No. 30 | |
| mouse 1F9D4 | No. 31 | |
| human 1B4G4 | | |
| human 1E7G5 | No. 33 | No. 34 |
| human 1E9F7 | | |
| human 1F9D4 | | |

2-2. Result

2-2-1. Cloning of anti-KMC07 antibody variable regions VH, VL

First, total RNA was extracted from 4 clones of hybridoma for the anti-KMC07 antibody, and using the extracted total RNA as a template, cDNA of each of the heavy chain and light chain variable regions VH and VL was synthesized. Next, PCR was performed using the cDNA as a template to amplify VH and VL genes of each clone. The results of agarose electrophoresis of the variable regions are shown in Fig. 3. At this time, PCR products amplified using primer sets 4 to 9 were defined as VH A to F, respectively. As a result, bands could be confirmed at a target position (0.4 to 0.5 kb) for 5 samples of A to E for 1B4G4 VL, 1 sample of C for 1B4G4 VH, 4 samples of B, C, E, and G for 1E7G5 VL, 1 sample of C for 1E7G5 VH, 5 samples of A to E for 1E9F7 VL, 1 sample of E for 1E9F7 VH, 2 samples of B and C for 1F9D4 VL, and C for 1F9D4 VH. Only a sample from which a band was obtained at the target position by agarose gel electrophoresis was selected for TA cloning. Thereafter, samples satisfying the following two conditions were selected: (1) the amplified PCR product was not a Sp2-derived gene; and (2) no mutation had been introduced in the course of PCR. Regarding (1), it was confirmed by colony PCR that the gene other than the inserted gene was not amplified. Regarding (2), for each sample, when a plurality of colonies were subjected to PCR and sequence alignment was performed, it was confirmed that there was no difference in the inserted gene sequence between the colonies. As a result, an antibody expression vector was constructed using gene fragments of A for 1B4G4 VL, C for 1B4G4 VH, G for 1E7G5 VL, C for 1E7G5 VH, A for 1E9F7 VL, E for 1E9F7 VH, C for 1F9D4 VL, and C for 1F9D4 VH.

Based on the cloning results, the amino acid sequences and base sequences of the variable regions in the heavy chain and the light chain of each anti-KMC07 antibody are shown in the following Tables 5A to 5D. In the following Tables, the amino acid sequence and the base sequence of the signal region (SP) in each of the heavy chain and the light chain are also shown.

[Table 5A]

| 1B4G4 (Antibody I) | | |
|---|---|---|
| Region in heavy chain | Variable | QVQLKESGPGLVQPSQTLSLTCTVSGFSLSSYGVIWVRQPPGKGLEWMGLIWDNGNTNYNSALKS RLSISRDTSKSQVFLKMNNLQTEDTAMYFCARSRHYFDYWGQGVMVTVSS (SEQ ID NO: 1) |
| | | CAGGTGCAGCTGAAGGAGTCAGGACCTGGCCTGGTGCAGCCCTCACAGACCCTGTCTCTCACC TGCACTGTCTCTGGGTTCTCACTAAGCAGCTATGGTGTAATCTGGGTTCGCCAGCCTCCAGGAA AGGGTCTGGAGTGGATGGGACTAATATGGGATAATGGAAATACAAATTATAATTCAGCTCTCA AATCCCGACTGAGCATCAGCAGGGACACCTCCAAGAGCCAAGTTTTCTTAAAAATGAACAATCT GCAAACTGAAGACACAGCCATGTACTTCTGTGCCAGATCGAGGCACTACTTTGATTACTGGGG CCAAGGAGTCATGGTCACAGTCTCCTCAG (SEQ ID NO: 9) |
| | Signal | MAVLVLLLCLVTLPSCALS (SEQ ID NO: 17) |
| | | ATGGCTGTCCTAGTGCTGCTCCTCTGCCTGGTGACACTTCCAAGCTGTGCCCTGTCC (SEQ ID NO: 18) |
| Region in light chain | Variable | DIQMTQSPSLLSASVGDRVTLNCKASQNIYKILAWYQQKLGEAPKLLIYNADSLQTGIPSRFSGSGS GTDFTLTISSLQPEDVATYFCQQYYSGWTFGGGTKLELK (SEQ ID NO: 2) |
| | | GACATCCAGATGACCCAGTCTCCTTCACTCCTGTCTGCATCTGTGGGAGACAGAGTCACTCTCA ACTGCAAAGCAAGTCAGAATATTTATAAGATCTTAGCCTGGTATCAGCAAAAGCTTGGAGAAGC TCCCAAACTCCTGATTTATAATGCAGACAGTTTGCAAACGGGCATCCCATCAAGGTTCAGTGGC AGTGGATCTGGTACAGATTTCACACTCACCATCAGCAGCCTGCAGCCTGAAGATGTTGCCACAT ATTTCTGCCAGCAGTATTATAGCGGGTGGACGTTCGGTGGAGGCACCAAGCTGGAATTGAAAC (SEQ ID NO: 10) |
| | Signal | MAPVQLLGLLVLFLPAMRC (SEQ ID NO: 19) |
| | | ATGGCTCCAGTTCAACTTTTAGGGCTTTTGGTGCTCTTCCTCCCAGCCATGAGATGT (1B4SP; SEQ ID NO: 20) |

[Table 5B]

| 1E7G5 (Antibody I) | | |
|---|---|---|
| Region in heavy chain | Variable | QVQLKESGPGLVQPSQTLSLTCTVSGFSLSSYGVIWVRQPPGKGLEWMGLIWDNGDTNYCSPLKS RLSISRDTSKSQVFLKMNNLQTEDTAMYFCARSRHYFDYWGQGVMVTVSS (SEQ ID NO: 3) |
| | | CAGGTGCAGCTGAAGGAGTCAGGACCTGGCCTGGTGCAGCCCTCACAGACCCTGTCTCTCACC TGCACTGTCTCTGGGTTCTCACTAAGCAGCTATGGTGTAATCTGGGTTCGCCAGCCTCCAGGAA AGGGTCTGGAGTGGATGGGACTAATATGGGATAATGGAGATACAAATTATTGTTCACCTCTCA AATCCCGACTGAGCATCAGCAGGGACACCTCCAAGAGCCAAGTTTTCTTAAAAATGAACAATCT GCAAACTGAAGACACAGCCATGTACTTCTGTGCCAGATCGAGGCACTACTTTGATTACTGGGG CCAAGGAGTCATGGTCACAGTCTCCTCAG (SEQ ID NO: 11) |
| | Signal | MAVLVLFLCLVTLPSCALS (SEQ ID NO: 21) |
| | | ATGGCTGTCTTAGTGCTGTTCCTCTGCCTGGTGACACTTCCAAGCTGTGCCCTGTCC (SEQ ID NO: 22) |
| Region in light chain | Variable | DIQMTQSPSLLSASVGDRVTLNCKASQNIYKNLAWYQQKLGEAPKFLIYNANSLQTGIPSRFSGSG SGTDFTLTISSLQPEDVATYFCQQYYSGWTFGGGTKLELK (SEQ ID NO: 4) |
| | | GACATCCAGATGACCCAGTCTCCTTCACTCCTGTCTGCATCTGTGGGAGACAGAGTCACTCTCA ACTGCAAAGCAAGTCAGAATATTTATAAGAACTTAGCCTGGTATCAGCAAAAGCTTGGAGAAG CTCCCAAATTCCTGATTTATAATGCAAACAGTTTGCAAACGGGCATCCCATCAAGGTTCAGTGG CAGTGGATCTGGTACAGATTTCACACTCACCATCAGCAGCCTGCAGCCTGAAGATGTTGCCAC ATATTTCTGCCAGCAGTATTATAGCGGGTGGACGTTCGGTGGAGGCACCAAGCTGGAATTGAA AC (SEQ ID NO: 12) |
| | Signal | MKLPVRLLVLFLPAMRC (SEQ ID NO: 23) |
| | | ATGAAGTTGCCTGTTAGGCTGTTGGTGCTCTTCCTCCCAGCCATGAGATGT (1E7SP; SEQ ID NO: 24) |

[Table 5C]

| 1E9F7 (Antibody II) | | |
|---|---|---|
| Region in heavy chain | Variable | QVQLQQSGAELVKPGSSVKISCKASGYTFTSNFMHWIKQQPGNGLEWIGWISPGDGDTEYNQKF NGKATLTADKSSSTAYMQLSSLTSEDSAVYFCARGGYYSGGGGYFDYWGQGVMVTVSS (SEQ ID NO: 5) |
| | | CAGGTACAGCTGCAGCAATCTGGGGCTGAACTGGTGAAGCCTGGGTCGTCAGTGAAAATTTCC TGCAAGGCTTCTGGCTACACCTTCACCAGTAACTTTATGCACTGGATAAAACAGCAGCCTGGAA ATGGCCTTGAGTGGATTGGGTGGATTTCTCCTGGAGATGGTGATACAGAGTACAATCAAAAGT TCAATGGGAAGGCAACACTCACTGCAGACAAATCCTCCAGCACAGCCTATATGCAGCTCAGCA GCCTGACATCTGAGGACTCTGCAGTCTATTTCTGTGCAAGAGGGGGGTACTACAGTGGTGGAG GGGGCTACTTTGATTACTGGGGCCAAGGAGTCATGGTCACAGTCTCCTCAG (SEQ ID NO: 13) |
| | Signal | MGWSCIMFFLVAAATCVHS (SEQ ID NO: 25) |
| | | ATGGGATGGAGCTGTATCATGTTCTTTCTGGTGGCAGCAGCTACATGTGTTCACTCC (SEQ ID NO: 26) |
| Region in light chain | Variable | DIVMTQSPTSMSISVGDRVTMNCKASQNLGSYVDWYQQKTGQSPKLLIYKASNRYTGVPDRFTGS GSGTDFTFSISNMQAEDLAVYYCMQSNSYLMYTFGAGTKLELK (SEQ ID NO: 6) |
| | | GACATTGTGATGACTCAGTCTCCCACATCCATGTCCATATCAGTAGGAGACAGGGTCACCATGA ACTGCAAGGCCAGTCAAAATTTGGGTTCTTATGTAGACTGGTACCAACAGAAAACAGGGCAGT CTCCTAAACTGCTTATCTACAAAGCATCCAACCGGTACACGGGAGTCCCTGATCGCTTCACAGG CAGTGGATCTGGAACAGATTTCACTTTCTCCATCAGCAACATGCAGGCTGAAGACCTGGCTGTT TATTACTGTATGCAGTCTAACTCCTATCTCATGTACACGTTTGGAGCTGGGACCAAGCTGGAAC TGAAAC (SEQ ID NO: 14) |
| | Signal | MESQTQVFIFLLLWLSGADG (SEQ ID NO: 27) |
| | | ATGGAGTCACAGACCCAGGTCTTCATATTCCTGCTGCTCTGGTTGTCTGGTGCTGATGGG (SEQ ID NO: 28) |

[Table 5D]

| 1F9D4 (Antibody III) | | |
|---|---|---|
| Region in heavy chain | Variable | EVQLVESGGGLVQPGRSLKLSCAASGFTFSDYNMAWVRQAPKKGLEWVATINYDGISTYYRDSVK GRFTISRDNAKSTLYLQMDSLRSEDTATYYCARHSYVYYALGVPFAYWGQGTLVTVSS (SEQ ID NO: 7) |
| | | GAGGTGCAGCTGGTGGAGTCTGGGGGAGGCTTAGTGCAGCCTGGAAGGTCCCTGAAACTCTCC TGTGCAGCCTCAGGATTCACTTTCAGTGACTATAACATGGCCTGGGTCCGCCAGGCTCCAAAG AAGGGTCTGGAGTGGGTCGCAACCATTAATTATGATGGTATTAGCACTTACTATCGAGACTCCG TGAAGGGCCGATTCACCATCTCCAGAGATAATGCAAAAAGCACCCTATACCTGCAAATGGACA GCCTGAGGTCTGAGGACACGGCCACTTATTACTGTGCTAGACATTCTTATGTATACTACGCATT AGGAGTCCCGTTTGCTTACTGGGGCCAAGGCACTCTGGTCACTGTCTCTTCAG (SEQ ID NO: 15) |
| | Signal | MDSRLNLVFLVLFMKGVQC (SEQ ID NO: 29) |
| | | ATGGACTCCAGGCTCAATTTAGTTTTCCTTGTCCTTTTTATGAAAGGTGTCCAGTGT (SEQ ID NO: 30) |
| Region in light chain | Variable | DIQMTQSPSLLSASVGDRVTINCKASQNINKYLNWYQQKLGEAPKLLIYNTNSLQTGIPSRFSGSG SGTDYTLTISSLQPEDFGTYFCFQHNSWPLTFGSGTKLEIK (SEQ ID NO: 8) |
| | | GACATCCAGATGACCCAGTCTCCTTCACTCCTGTCTGCATCTGTGGGAGACAGAGTCACTATCA ACTGCAAAGCAAGTCAGAATATTAACAAGTACTTAAACTGGTATCAGCAAAAGCTTGGAGAAG CTCCCAAACTCCTGATATATAATACAAACAGTTTGCAAACGGGCATCCCATCAAGGTTCAGTGG CAGTGGATCTGGTACAGATTACACACTCACCATCAGCAGCCTGCAGCCTGAAGATTTTGGCAC ATATTTCTGCTTTCAGCATAATAGTTGGCCGCTCACGTTCGGTTCTGGGACCAAGCTGGAGATC AAAC (SEQ ID NO: 16) |

## 2-2-2. Construction of anti-KMC07 antibody expression vector

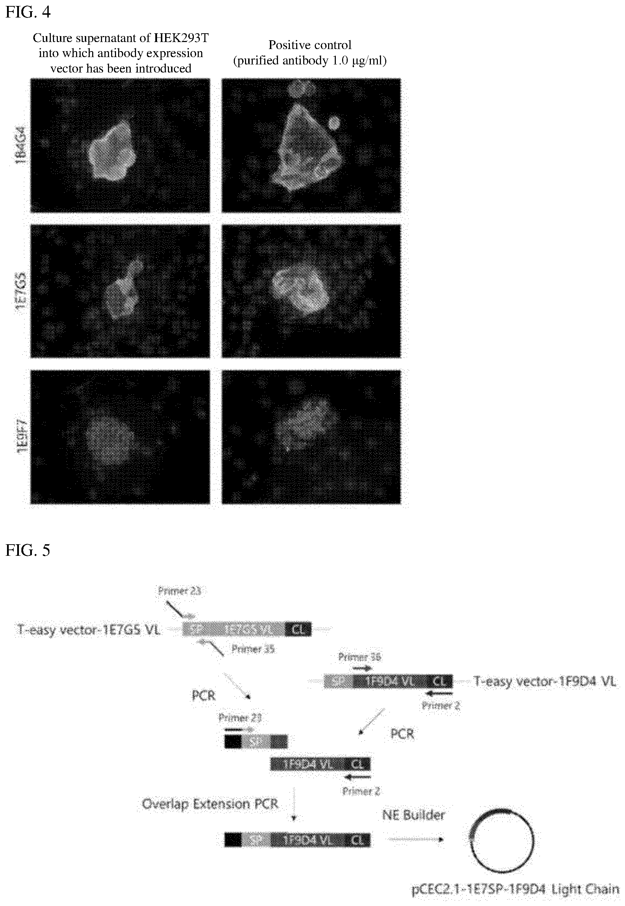

Variable region genes of 4 clones, 1B4G4 (antibody I), 1E7G5 (antibody I), 1E9F7 (antibody II), and 1F9D4 (antibody III), were amplified by PCR using, as a template, a cloning vector incorporating a polynucleotide composed of the base sequences of the variable regions and the signal regions (SP) shown in Tables 5A to 5D. Thereafter, the amplified genes were inserted into animal expression vectors pCEC2.1-rIgG2a and pCEC2.1-rIgK incorporating heavy chain and light chain constant region genes using NE builder. After confirming that there was no problem in the gene sequence by sequencing, each of the produced anti-KMC07 antibody expression vectors was introduced into HEK293T. Immunostaining of KMC07 was performed using the culture supernatant of each cell. In this immunostaining, cell nuclei are stained blue and each antibody is stained green. The results are shown in Fig. 4. As shown in Fig. 4, signals were obtained on the cell membrane of KMC07 for 1B4G4 (antibody I), 1E7G5 (antibody I), and 1E9F7 (antibody II), so that expression of 3 clones of the anti-KMC07 antibody could be confirmed. As a positive control, an antibody purified from the hybridoma culture supernatant was used.

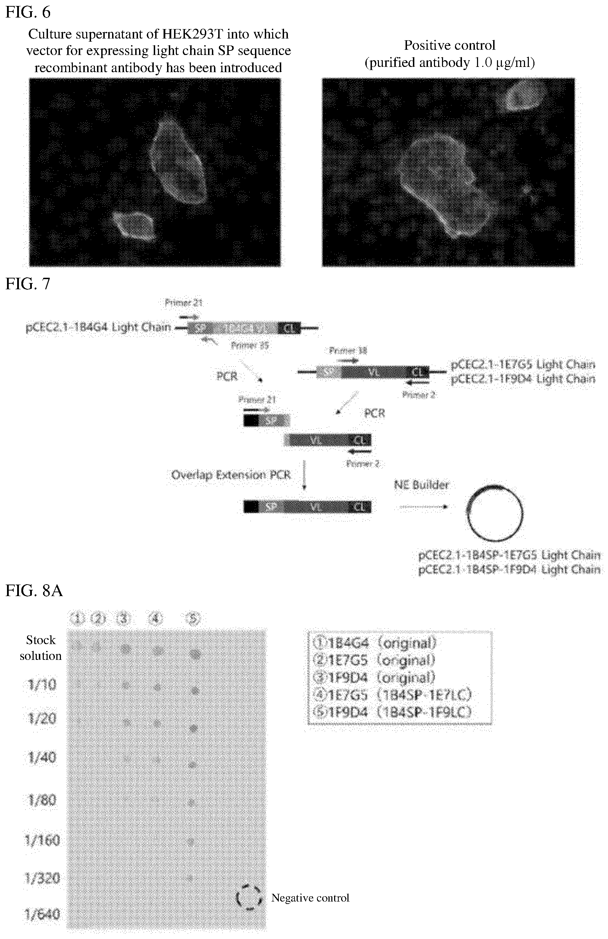

On the other hand, no signal was obtained with 1F9D4 (antibody III). Since the SP sequence of 1F9D4VL was not recognized as an SP sequence on the amino acid domain database SMART, it was considered that there was some problem in the SP sequence. Accordingly, a 1E7SP-1F9D4 light chain expression vector was produced in which the SP sequence of the light chain of 1E7G5 (antibody I) (1E7SP; SEQ ID NO: 24) had been recombined with the light chain of 1F9D4 (antibody III). The flow of construction for the 1E7SP-1F9D4 light chain expression vector is shown in Fig. 5. The produced 1E7SP-1F9D4 light chain expression vector and 1F9D4 heavy chain expression vector were introduced into HEK293T, followed by culture. The culture supernatant was immunostained for KMC07. The results are shown in Fig. 6. As shown in Fig. 6, a signal was obtained on the cell membrane of KMC07. Therefore, it is considered that by recombining the SP sequence of the light chain with that of 1E7G5 (SEQ ID NO: 24), the amount of 1F9D4 (antibody III), which is an anti-KMC07 antibody, secreted into the culture supernatant increased.

2-2-3. Construction of anti-KMC07 human chimeric antibody and mouse chimeric antibody expression vectors

PCR using an anti-KMC07 antibody heavy chain expression vector as a template was performed to amplify the VHCH1-Hinge gene fragment of each clone. Thereafter, the amplified gene fragments were inserted into animal expression vectors pCEC2.1-hIgGl and pUC19-mIgG2a incorporating human and mouse Fc-region genes using NE builder. After confirming that there was no problem in the gene sequence by sequencing, each of the produced anti-KMC07 antibody human chimeric heavy chain expression vector and mouse chimeric heavy chain expression vector was introduced into Exp293F together with a light chain expression vector. When the chimeric antibody was purified from the culture supernatant, chimeric antibodies of 1B4G4 (antibody I) and 1E9F7 (antibody II) could be obtained in a sufficient antibody amount.

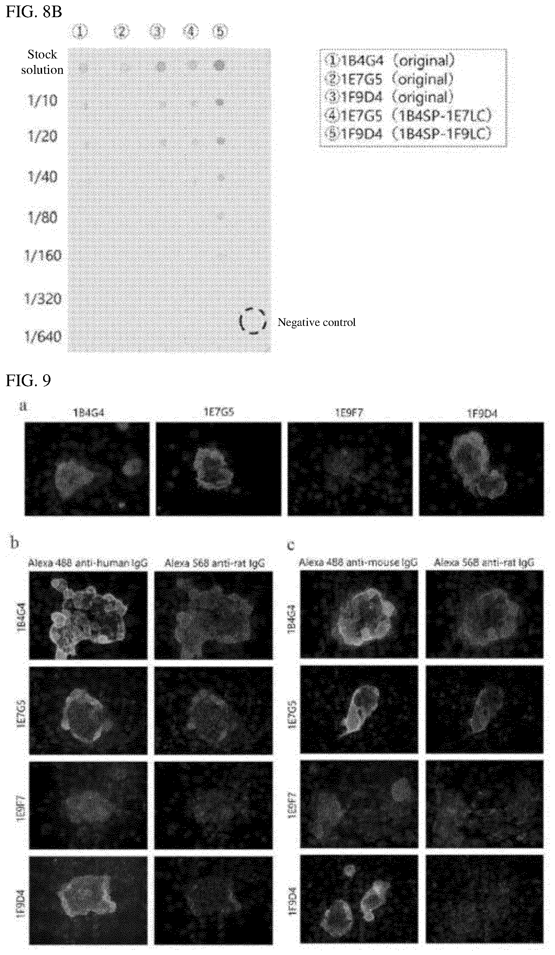

On the other hand, chimeric antibodies of 1E7G5 (antibody I) and 1F9D4 (antibody III) were hardly obtained. Accordingly, among the above 2 clones from which a sufficient antibody amount was obtained, a 1B4SP-1E7G5 light chain expression vector and a 1B4SP-1F9D4 light chain expression vector (hereinafter, also collectively referred to as "1B4SP-added light chain vector") were produced in which the light chain SP sequence of 1B4G4 (antibody I) sequence (1B4SP; SEQ ID NO: 20) similar to the light chain SP sequence (SEQ ID NO: 24) of 1E7G5 (antibody I) had been recombined with the light chains of 1E7G5 (antibody I) and 1F9D4 (antibody III), respectively. The flow of construction for the 1B4SP-added light chain vector is shown in Fig. 7. The produced 1B4SP-added light chain vector, rat light chain expression vector, rat heavy chain expression vector, and mouse chimeric heavy chain expression vector were introduced into Expi293F, followed by culture. By serially diluting the culture supernatant and performing dot blot, the amount of each antibody secreted into the culture supernatant was compared. As a positive control, a culture supernatant of Expi293F into which a 1B4G4 expression vector had been introduced was used. The results of confirming the color developing limit are shown in Fig. 8A (Expi293F culture supernatant into which a rat antibody expression vector has been introduced) and Fig. 8B (Expi293F culture supernatant into which a mouse chimeric antibody expression vector has been introduced). As shown in Figs. 8A and 8B, in both the rat antibody and the mouse chimeric antibody produced using the 1B4SP-added light chain vector, the antibody amount increased about 8-fold for 1E7G5 (antibody I) and about 4-fold for 1F9D4 (antibody III) as compared with the original antibody. Since it could be confirmed that both of them were equal to or more than the antibody amount of 1B4G4 (antibody I) used as a positive control, chimeric antibodies of 1E7G5 and 1F9D4 were purified from the culture supernatant of Expi293F. By thus recombining the light chain signal sequence with SEQ ID NO: 20, a sufficient amount of chimeric antibodies of 1E7G5 (antibody I) and 1F9D4 (antibody III) could be obtained.

Immunostaining of KMC07 was performed using the produced antibody. As a positive control, an antibody purified from the hybridoma culture supernatant was used. In this immunostaining, cell nuclei are stained blue, and each antibody is stained green (Alexa 488) or red (Alexa 568). The results are shown in Fig. 9 (a: rat antibody, b: rat/human chimerized antibody, c: rat/mouse chimerized antibody). As a result, signals similar to that of the original rat antibody were obtained in all the clones. From the above, it can be said that rat/human chimerization and rat/mouse chimerization of 4 clones of the anti-KMC07 antibody were successful.

Test Example 3: Characterization of monoclonal antibody against KMC07

3-1. Materials and methods

Cell culture

As NK92/CD16a (NK92 (human natural killer cell line) by which CD16a had been forcibly expressed), 100 U/ml human IL-2 [Shionogi & Co., Ltd., Immunace (registered trademark)]-containing MyeloCultTM H5100 [STEMCELL, 05150] was used, followed by culture at 37°C under 5% by volume $CO_2$. The method for culturing other cells was according to "Cell culture" and "Culture of KMC07" in "1-1. Materials and methods" of Test Example 1.

Immunoprecipitation

To an extract (protein amount: 600 $\mu$g) prepared using a membrane protein extract preparation kit (Mem-PERTM Plus Kit [Thermo fisher, 89842Y]) were added 25 $\mu$l of beads, followed by stirring at 4°C for 30 minutes. After removing the supernatant, washing was performed 5 times with RIPA buffer, followed by addition of 20 $\mu$l of a 2 × Sample buffer (0.125 M Tris-HCl, 4% SDS, 10% sucrose, 0.1% bromophenol blue, 10% 2-mercaptoethanol) solution, and then incubation at 98°C for 5 minutes. The eluted proteins were subjected to SDS gel electrophoresis (SDS-PAGE) using Hybrid Gel, and then evaluated by silver staining (2D-silver staining reagent [Cosmo Bio, 423413]).

Western blotting analysis

A membrane was prepared using the immunoprecipitate as an antigen. The other methods are according to the "Western blotting analysis" in "1-1. Materials and methods" of Test Example 1.

Mass analysis

A band obtained by silver staining the immunoprecipitate was cut out and then subjected to silver removal for subsequent mass spectrometry.

Purification of binding protein using antibody affinity column

The purified anti-KMC07 antibody was coupled to 1 ml of HiTrap NHSactivated HP [GE Healthcare, 17-0716-01]. Next, KMC07 in 30 P10 dishes cultured until it became confluent was collected by a scraper. After centrifugation at 1,200 rpm for 10 min at 4°C, the supernatant was removed and KMC07 cell suspension was prepared in RIPA-buffer (1 tablet of ULTRARIPAR Kit for Lipid Raft [BDL, F015] per 20 ml of 5 $\mu$g/ml Aprotinin, 5 $\mu$g/ml Leupeptin, 20 $\mu$g/ml APMSF, cOmplete (trademark) Protease Inhibitor Cocktail [Roche, 04693116001]). The KMC07 cell suspension was applied to an antibody coupling column by a peristaltic pump, followed by circulation at 4°C for 1 hour. The solution flowed out at this time was applied to a column coupled with another anti-KMC07 antibody, followed by circulation at 4°C for 1 hour. This was repeated for 5 clones of the anti-KMC07 antibody for antibody and protein reaction. Finally, proteins were eluted with an elution buffer (pH 3.0, 1% Glycine, 1% n-octyl-$\beta$-D-glucoside [Dojindo, 346-05033]) for SDS-PAGE using 12.5% polyacrylamide gel, followed by evaluation by silver staining (ProteoSilver (trademark) plus silver staining kit [SIGMA, PROT-SIL2]).

Introduction of protein expression vector into HEK293T and confirmation of introduction

To 80 $\mu$l of FBS-free D-MEM was added 1 $\mu$g of the protein expression vector. To this, 3 $\mu$l of 1 mg/ml PEI MAX was added, and the mixture was allowed to stand at room temperature for 20 minutes. Then, 1 ml of 2% FBS-containing D-MEM was added. The culture supernatant of HEK293T cultured in a 12 well plate was removed, and a prepared solution was added. One day after incubation at 37°C under 5% CO2, assessment was made by immunostaining. The immunostaining was according to "Immunostaining" in "1-1. Materials and methods" of Test Example 1. An anti-EpCAM rabbit polyclonal antibody [proteintech, 21050-1-AP] was diluted 100 times for use as a positive control antibody.

Case where cell membrane is perforated for immunostaining: Cells were fixed by treatment with a 3.7% solution of formaldehyde in PBS for 15 minutes. The cell was treated with a 0.5% solution of Triton X-100 in PBS for 5 minutes, and then the cell membrane was perforated. Blocking was performed in a solution of NGS blocking (100 mg/ml BSA, Goat Serum [Gibco, 16210-064], 37.5 mg/ml glycine [Wako, 077-00735], 0.1% sodium azide [Wako, 197-11091]) in PBS for 30 minutes. After removal of the solution, a primary antibody was added, followed by incubation for 1 hour.

Furthermore, incubation was performed with a solution of a secondary antibody (Alexa 488 anti-rat IgG (H+L)) in PBS containing Hoechst (registered trademark) 33342 for 30 minutes. Finally, a discoloration inhibitor DABCO was added, followed by observation with a fluorescence microscope.

Assessment of ADCC activity (LDH assay method, vs. BxPC3)

In order to measure the ADCC (Antibody Dependent Cellular Cytotoxicity) activity of the anti-KMC07 antibody 1F9D4 (antibody III) against BxPC3 (pancreatic adenocarcinoma cell line) in the presence of effector cells, the activity of LDH (lactate dehydrogenase) released from the cells was measured using Cytotoxicity LDH Assay Kit-WST [DOJINDO, CK12]. The medium for effector cells (NK92/CD16a) was changed 18 hours before the start of the assay. Target cells (BxPC3) were seeded at $5.0 \times 10^3$ cells/well in a 96 well plate 16 hours before the start of the assay, followed by culture at 37°C under 5% by volume $CO_2$. On the day of the assay, the effector cells and the anti-KMC07 rat/human chimerized antibody 1F9D4 were diluted with Assay Buffer (1% FBS-containing RPMI-1640) so as to be 2 times thicker than the final concentration, and seeded at 50 μl/well in a 96 well plate from which the culture supernatant had been removed, followed by incubation at 37°C for 4 hours under 5% by volume $CO_2$ (n = 3). At this time, as negative controls, wells for culturing only target cells, only effector cells, and wells of only 100 μl of Assay Buffer, only Lysis solution (91 μl of Assay Buffer, 9 μl of Lysis Solution) were made. Furthermore, 45 minutes before the end of incubation, 9 μl of Lysis solution was added to the target cells cultured in 91 μl of Assay buffer as a positive control. After 4 hours, the 96 well plate was centrifuged at 200 g for 4 minutes, and 50 μl each of the culture supernatants was transferred to a new 96 well plate. To this was added 50 μl each of a substrate solution (Dye Mixture-containing Assay Buffer), followed by reaction at room temperature for 15 to 30 minutes under a light-shielded condition. Finally, 50 μl each of Stop Solution was added to stop the reaction, and then the absorbance at 490 nm was measured. The absorbance for a well in which effector cells and an antibody were added to target cells was denoted as A, the absorbance for a well containing only effector cells was denoted as B, the absorbance for a well containing only target cells was denoted as C, and the absorbance for a well in which Lysis Solution was added to kill target cells was denoted as D, and the ADCC activity was calculated from the following calculation formula. For each absorbance, a value obtained by subtracting the background absorbance of each solution alone was used.

[Mathematical formula 1]

$$ADCC\,(\%) = \{(A\text{-}B\text{-}C)/(D\text{-}C)\} \times 100$$

Assessment of ADCC activity (Colony assay method, vs. KMC07)

In order to measure the ADCC activity of the anti-KMC07 antibody against KMC07 in the presence of effector cells, the number of colonies of KMC07 was counted over time. Hereinafter, this experimental system is referred to as a colony assay. In a 12 well plate in which glass coated with 1.5 μg/ml poly-L-ornithine 3 days before the start of the assay was immersed, PA6 (mouse fibroblast cell line) was seeded at $5.0 \times 10^4$ cells/well and cultured at 37°C under 5% by volume $CO_2$. The medium for effector cells (NK92/CD16a) was changed 18 hours before the start of the assay. Target cells (KMC07) were seeded at $5.0 \times 10^4$ cells/well in a 12 well plate 16 hours before the start of the assay, followed by culture at 37°C under 5% by volume $CO_2$. On the day of the assay, effector cells were diluted with KMC Medium (whose composition is described in "Culture of KMC07" in "1-1. Materials and methods" of Test Example 1) to an appropriate concentration, and anti-KMC07 rat/human chimerized antibodies (1B4G4 (antibody I) and 1F9D4 (antibody III)) were diluted therewith to 1 μg/well, and seeded at 500 μl/well in a 12 well plate from which the culture supernatant had been removed, followed by incubation at 37°C under 5% by volume $CO_2$ (n = 2). At this time, assuming that KMC07 was present at about $5.0 \times 10^4$ cells/well, which was half the amount of PA6, effector cells were seeded at $2.5 \times 10^5$ cells/well in order to set the E : T ratio = 5 : 1. After 6 hours, 12 hours, and 24 hours, immunostaining was performed to confirm colonies of KMC07. After treatment with a 3.7% solution of formaldehyde in PBS for 15 minutes to fix the cells, an anti-KMC07 rat antibody 1B4G4 (antibody I) (1 μg/ml) was used as a primary antibody for incubation for 1 hour. Furthermore, incubation was performed with a solution of a secondary antibody (Alexa 488 anti-rat IgG (H+L)) in PBS containing Hoechst (registered trademark) 33342 for 30 minutes. Finally, a discoloration inhibitor DABCO was added, followed by observation with a fluorescence microscope. For each glass, 9 sites were randomly selected, and the number of KMC07 colonies was measured.

Production of tumor-bearing mouse

On the backs of 15 BALB/cSlc-nu/nu nude mice [SLC, 6-weeks old female], 100 μl each of a KMC07 + PA6 cell suspension ($3.5 \times 10^7$ cells/ml) suspended in 10% FBS-containing MEMα was subcutaneously injected.

In vivo evaluation of antitumor effect using tumor-bearing mice

Mice were grouped into groups of 3 per test antibody depending on tumor size 3 weeks after production of tumor-bearing mouse (n = 3). Each of the anti-KMC07 antibodies 1E7G5 (antibody I), 1F9D4 (antibody III), and the negative control antibody 4G8B4 that is not an anti-KMC07 antibody was injected into the tail vein once a week in a volume of 200 µl of a solution of 0.25 mg/ml anti-KMC07 rat/mouse chimeric antibody in PBS. This operation was performed six times in total. The body weight and tumor diameter of each mouse were measured by caliper twice a week. The tumor volume was estimated from the following calculation formula.

[Mathematical formula 2]

$$\text{Tumor volume (mm}^3) = \{\text{major axis} \times (\text{minor axis})^2\}/2$$

Statistical analysis

All statistical analyses were performed by two-way ANOVA.

3-2. Result

3-2-1. Search for antigen molecule by immunoprecipitation using KMC07

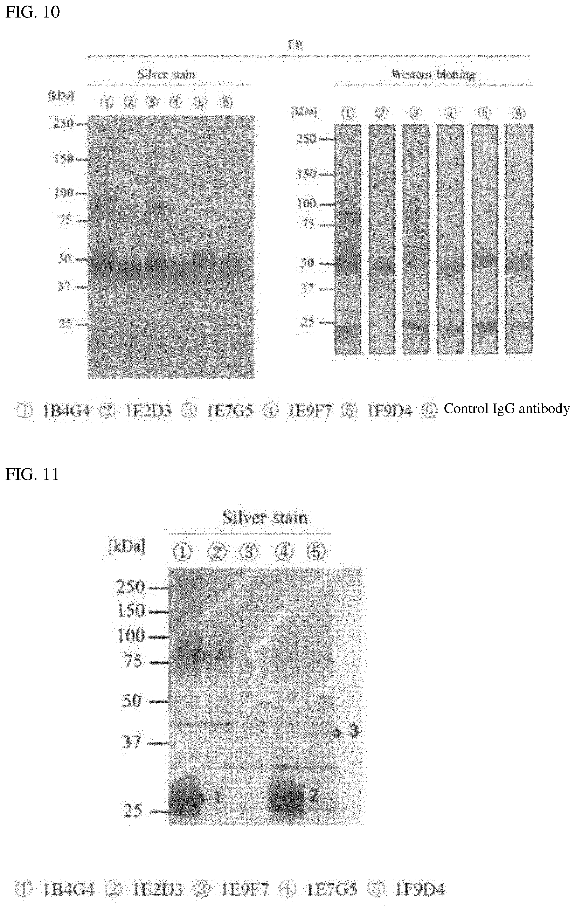

Immunoprecipitation using a membrane protein extract was performed on solubilized proteins with denaturation suppressed as much as possible. The results of performing silver staining on the KMC07 membrane protein extract using 5 clones of the KMC07 antibody and 10A7 as a control IgG antibody are shown in Fig. 10.
For 1B4G4 and 1E7G5 (antibody I), broad bands (indicated by the arrow in the figure) were obtained between 75 and 100 kDa, and for 1F9D4 (antibody III), bands (indicated by the arrow in the figure) were obtained near 140 kDa and 37 kDa. The band pattern of silver staining and the result of "1-2-2. Reactivity against various human cancer cell line" in "1-2. Result" of Test Example 1 suggested that 1B4G4 and 1E7G5 (antibody I) may recognize the same antigen.

3-2-2. Search for antigen molecule using antibody binding column

To an affinity column, 5 clones of the anti-KMC07 antibody were bound and the KMC07 whole cell extract was flown to react the antibody with the antigen. Thereafter, the antibody was eluted and evaluated by silver staining. The results are shown in Fig. 11. As shown in Fig. 11, a band specific to 75 to 100 kDa (mark 4 in the figure) for 1B4G4 (antibody I), bands specific near 25 kDa (marks 1 and 2 in the figure) for 1B4G4 and 1E7G5 (antibody I), and a band specific to 37 kDa (mark 3 in the figure) for 1F9D4 (antibody III) were obtained. Among them, the broad band of 75 to 100 kDa (mark 4 in the figure) detected with 1B4G4 was similar to that in Fig. 10, and thus considered to be promising as a candidate antigen. Each band was cut out for mass analysis, and the results are summarized in Table 6.

[Table 6]

| Sample | | Result | MW(Da) |
|---|---|---|---|
| 1B4G4 75-100 kDa | → | Olfactomedin-4 | 57244 |
| 1B4G4 25 kDa | → | 14-3-3 protein beta/alpha | 28065 |
| 1E7G5 25 kDa | → | 14-3-3 protein beta/alpha | 28065 |
| 1F9D4 37 kDa | → | EpCAM | 34910 |

As shown in Table 6, 14-3-3 protein beta/alpha (14-3-3) was hit for 1B4G4 and 1E7G5 (antibody I). However, since 14-3-3 is a protein involved in signal transduction in a cell and is known to interact with various proteins, it is considered that 14-3-3 non-specifically bound to the antibody. On the other hand, Olfactomedin-4 (OLFM4) detected with 1B4G4 (antibody I), which is a secreted protein and has a molecular weight of about 57 kDa, is used as a candidate antigen of 1B4G4 (antibody I) from the fact that the Olfactomedin is (1) known as an intestinal epithelial stem cell marker, (2) known to be widely expressed in gastric cancer and colorectal cancer, and be involved in the progression of cancer, (3) known that the molecular weight becomes 72 kDa when the sugar chain is modified, and (4) known to interact with cadherin present in a cell membrane. It is likely that OLFM4 is subjected to special modification such as specific sugar chain modification by KMC07 and is anchored to the cell membrane. Furthermore, EpCAM detected by 1F9D4 (antibody III) is a type I transmembrane glycoprotein, is known to be involved in not only cell adhesion but also signal transduction and cell migration, is expressed in many cancer types, and has recently attracted attention as a cancer stem cell marker. Considering that 1F9D4 (antibody III) also reacts with cancer cell lines other than KMC07, it is determined that 1F9D4 (antibody III) is likely to recognize EpCAM, and thus EpCAM was used as a candidate antigen of 1F9D4 (antibody III).

3-2-3. Reactivity against candidate antigen molecule

HA-OLFM4 and EpCAM expression vectors were each introduced into HEK293T, and the reactivity of 5 clones of the anti-KMC07 antibody was evaluated by immunostaining. Commercially available anti-HA and anti-EpCAM antibodies were used as positive control antibodies, and each antibody was reacted with HEK293T untreated as a negative control. As a result, only for 1F9D4 (antibody III), a signal was obtained in HEK293T by which EpCAM had been forcibly expressed. Therefore, the antigen of 1F9D4 (antibody III) was more likely EpCAM.

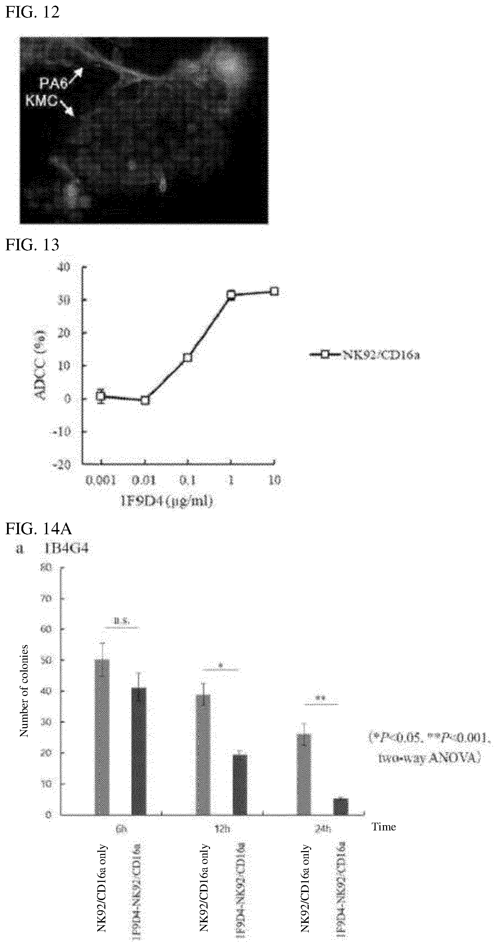

Since it was considered that EpCAM was so specially modified with KMC07 that its expression pattern was changed, expression of EpCAM was confirmed using alive or fixed Triton X-100-treated KMC07 by immunostaining. At this time, the anti-KMC07 antibody 1F9D4 and a commercially available EpCAM antibody were used as primary antibodies. When KMC07 was immunostained as living cells, signals were obtained on the cell membrane of KMC07 and the cell membrane of PA6 with the anti-EpCAM antibody (indicated by the arrow in Fig. 12). The staining pattern of KMC07 was similar to that with 1F9D4. Since the anti-EpCAM antibody is known to cross human, mouse and rat EpCAMs, it is considered that the anti-EpCAM antibody also reacted to the mouse fibroblast cell line PA6. On the other hand, 1F9D4 (antibody III) did not react with PA6 but reacted only with KMC07, suggesting that 1F9D4 (antibody III) specifically recognizes a human EpCAM.

3-2-4. In vitro evaluation of ADCC activity against BxPC3

The cytotoxicity was measured by measuring the LDH (lactate dehydrogenase) released from cells. Specifically, ADCC activity against BxPC3 was evaluated for 1F9D4 that also reacts with cancer cells other than KMC07. Here, the human chimeric antibody 1F9D4 produced in Test Example 2 was used.

First, it was examined at what ratio (E : T ratio) NK92/CD16a as an effector cell was reacted with BxPC3 as a target cell to determine whether 1F9D4 exhibits ADCC activity. 1F9D4 was used at an antibody concentration of 1 $\mu$g/ml. As a result, it was confirmed that the cytotoxicity of about 40% was exhibited at the E : T ratio of 5 : 1, and the dead cell percentage did not increase even when the E : T ratio was increased any more. That is, since it was found that 1F9D4 exhibited sufficient ADCC activity at an E : T ratio of 5 : 1, the subsequent experiments were performed at an E : T ratio of 5 : 1. Next, the test was performed by changing the concentration condition of 1F9D4. NK92/CD16a was used as an effector cell. The results are shown in Fig. 13. As shown in Fig. 13, when NK92/CD16a was used as an effector cell, 1F9D4 exhibited ADCC activity in a concentration dependent manner from 0.01 $\mu$g/ml. From the above, it was confirmed that 1F9D4 exhibited ADCC activity against pancreatic cancer cells in vitro.

3-2-5. In vitro evaluation of ADCC activity against KMC07

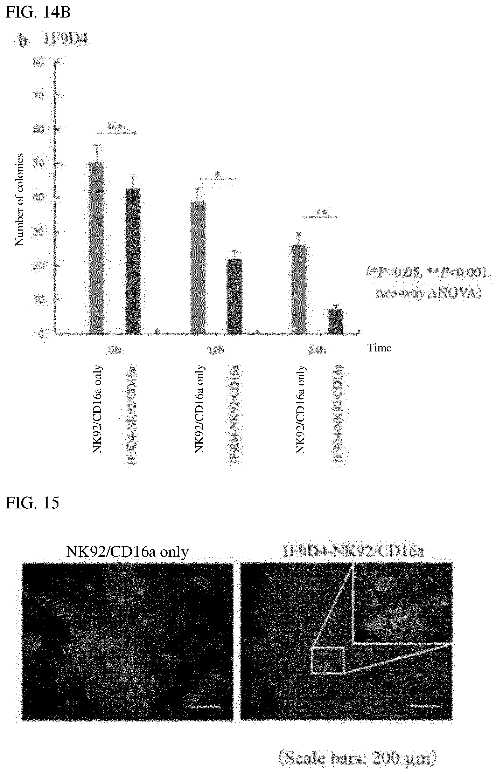

ADCC activity was evaluated by measuring the number of colonies of KMC07 at 6, 12 and 24 hours after addition of effector cells and an anti-KMC07 rat/human chimeric antibody. As the antibody, 1B4G4 (antibody I) and 1F9D4 (antibody III) diluted to 1 $\mu$g/ml were used, and the E : T ratio was 5 : 1. The results are shown in Figs. 14A (1B4G) and 14B (1F9D4). As shown in Figs. 14A and 14B, ADCC activity against KMC07 was observed by the anti-KMC07 antibodies 1B4G4 (antibody I) and 1F9D4 (antibody III) even thinking that the number of colonies decreased over time for the effect cell CD16a (KMC07 was considered to suffer some damage by relatively long-term co-culture with effector cells.), suggesting that the ADCC activity increased over time.

Since KMC07 proliferates while forming a colony, it is considered that the antibody hardly infiltrates the inside of the colony. Therefore, by increasing the reaction time of the antibody, it is considered that the antibody has succeeded in invading the inside while damaging KMC07 outside the colony, and dramatically reducing the colony. This was confirmed, since in the immunostained image of KMC07 24 hours after the reaction of 1F9D4 (antibody III) (Fig. 15), it was observed that the number of colonies of KMC07 was clearly smaller for 1F9D4-NK92/CD16a than that for only NK92/CD16a as a negative control, and that the cell membrane was broken in some of KMC07 for 1F9D4-NK92/CD16a (Fig. 5, enlarged view). Therefore, it was suggested that KMC07 was injured by ADCC activity of the anti-KMC07 antibody 1F9D4 (antibody III). The same result was obtained with the other clone 1B4G4 (antibody I).

3-2-6. In vivo evaluation of antitumor effect using tumor-bearing mice

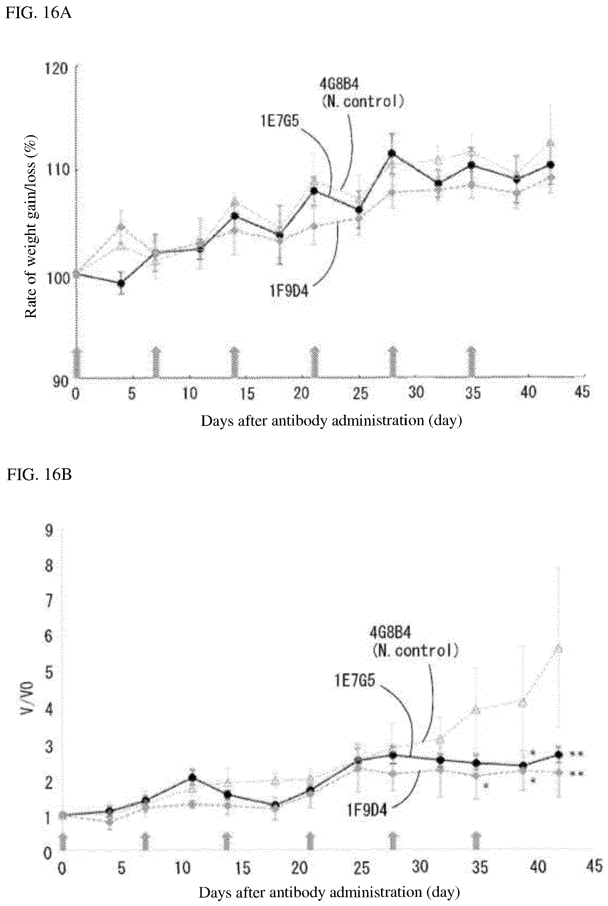

The anti-KMC07 antibody was administered to a tumor-bearing mouse produced using KMC07, and the tumor volume was measured over time to evaluate the antitumor effect. At this time, 2 clones of the anti-KMC07 mouse chimeric antibody 1E7G5 (antibody I) and 1F9D4 (antibody III) produced in Test Example 2 and an anti-Septin 7 mouse monoclonal antibody 4G8B4 produced in this laboratory were used as negative controls (hereinafter, described as control antibodies). Since 4G8B4 is an antibody against a protein expressed in cytoplasm, 4G8B4 is considered to be less toxic to mice. Each antibody was administered to 3 tumor-bearing mice by tail vein injection once a week for a total of six times (n = 3). The body weight and the tumor volume were measured twice a week, and the temporal change of the rate of weight gain/loss based on the start date of the clinical trial was shown in Fig. 16A. As shown in Fig. 16 A, since a large decrease in body weight due to administration of each antibody was not observed, it is considered that the antibody has no toxicity. In addition, based on the tumor volume ratio $V/V_0$ obtained by dividing each tumor volume V by the tumor volume $V_0$ at the time of the first antibody administration, the temporal change of the tumor volume ratio $V/V_0$ based on the start date of the clinical trial is shown in Fig. 16B. As shown in Fig. 16B, both 2 clones of the anti-KMC07 antibodies 1E7G5 (antibody I) and 1F9D4 (antibody III) had lower $V/V_0$ than that when the control antibody was administered. Statistically significant differences were observed with respect to the control antibody on days 35, 39, and 42 after the start of antibody administration for 1F9D4, and on days 39 and 42 after the start of antibody administration for 1E7G5 (day 35: 1F9D4 $P < 0.05$, day 39: 1E7G5 $P < 0.05$, 1F9D4 $P < 0.05$, day 42: 1E7G5 $P < 0.001$, 1F9D4 $P < 0.001$).

Furthermore, for each antibody, when multiple comparison of the tumor volume ratios for the respective measurement days was made by two-way ANOVA, for the anti-KMC07 antibodies 1E7G5 and 1F9D4, no significant difference in tumor volume ratio was found for the respective measurement days, while for the control antibody, a significant difference ($P < 0.05$) in that the tumor was enlarged between the measurement days as shown in Table 7 was found. Therefore, it was suggested that for the mice to which the anti-KMC07 antibodies 1E7G5 (antibody I) and 1F9D4 (antibody III) had been administered, the enlargement of tumor was suppressed from the initial stage of the clinical trial, whereas for the mice to which the control antibody had been administered, the tumor continued to be enlarged.

[Table 7]

Result of multiple comparison of tumor volume ratio $V/V_0$ for respective measurement days in 4G8G4

| pair (day) | r | nominal level | t | p | sig. |
|---|---|---|---|---|---|
| 42 - 0 | 13 | 0.0025641 | 6.693 | 0.0000000 | s. |
| 42 - 4 | 12 | 0.0027972 | 6.669 | 0.0000000 | s. |
| 42 - 7 | 11 | 0.0030769 | 6.262 | 0.0000000 | s. |
| 42 - 11 | 10 | 0.0034188 | 5.666 | 0.0000003 | s. |
| 42 - 14 | 9 | 0.0038462 | 5.386 | 0.0000009 | s. |
| 42 - 18 | 8 | 0.0043956 | 5.311 | 0.0000012 | s. |
| 42 - 21 | 7 | 0.0051282 | 5.265 | 0.0000014 | s. |
| 39 - 0 | 12 | 0.0027972 | 4.523 | 0.0000235 | s. |
| 42 - 25 | 6 | 0.0061538 | 4.512 | 0.0000245 | s. |
| 39 - 4 | 11 | 0.0030769 | 4.499 | 0.0000256 | s. |
| 35 - 0 | 11 | 0.0030769 | 4.242 | 0.0000649 | s. |
| 35 - 4 | 10 | 0.0034188 | 4.218 | 0.0000707 | s. |
| 39 - 7 | 10 | 0.0034188 | 4.092 | 0.0001102 | s. |
| 42 - 28 | 5 | 0.0076923 | 4.030 | 0.0001369 | s. |
| 35 - 7 | 9 | 0.0038462 | 3.811 | 0.0002891 | s. |
| 42 - 32 | 4 | 0.0102564 | 3.681 | 0.0004451 | s. |
| 39 - 11 | 9 | 0.0038462 | 3.496 | 0.0008117 | s. |
| 39 - 14 | 8 | 0.0043956 | 3.216 | 0.0019475 | s. |
| 35 - 11 | 8 | 0.0043956 | 3.215 | 0.0019528 | s. |
| 39 - 18 | 7 | 0.0051282 | 3.142 | 0.0024387 | s. |
| 39 - 21 | 6 | 0.0061538 | 3.095 | 0.0028020 | s. |
| 35 - 14 | 7 | 0.0051282 | 2.935 | 0.0044755 | s. |
| 35 - 18 | 6 | 0.0061538 | 2.860 | 0.0055337 | s. |
| 35 - 21 | 5 | 0.0076923 | 2.814 | 0.0063072 | s. |

From the above in vitro and in vivo results, it can be said that the produced antibody exhibited an antitumor effect.

Test Example 4: Use of monoclonal antibody against KMC07 as extracorporeal diagnostic agent

4-1. Materials and methods

Cell culture

The method for culturing cells was according to "Cell culture" and "Culture of KMC07" in "1-1. Materials and methods" of Test Example 1.

Purification of extracellular vesicle

Extracellular vesicles were purified from culture supernatants of KMC07 + PA6 and PA6 using ultracentrifugation. Ultracentrifugation was performed on 30 ml of culture supernatant at 110,000 rpm for 70 min at 4°C. The supernatant was removed, followed by suspension in 500 μl of PBS to prepare an extracellular vesicle fraction.

Dot blot

On a membrane (HybondTM-C Extra) was blotted 1 μl of a solution of extracellular vesicle in PBS. The membrane was air-dried and then immersed in a 3% solution of skim milk in TBS-T for 30 minutes. After blocking, a primary antibody diluted to 1 μg/ml with Can Get Signal Solution I was added, followed by incubation for 1 hour. After washing with TBS-T, the membrane was incubated for 30 minutes with a secondary antibody (Anti-Rat IgG-Peroxidase, antibody produced in rabbit [SIGMA, A5795-1ML]) diluted with Can Get Signal Solution II. Finally, color development was performed using KPL TMB Membrane Peroxidase Substrate [SeraCare, 5420-0027].

HRP labeling of antibody

As HRP labeling of the antibody, Ab-10 Rapid Peroxidase Labeling Kit [Dojindo, LK33] was used. After labeling, the antibody concentration was 0.83 mg/ml.

Color development detection sandwich ELISA

On an ELISA plate [Thermo Scientific, 439454] was seeded 100 μl of the anti-KMC07 antibody prepared to be 10 μg/ml as a capture antibody, followed by incubation at 4°C overnight. After rinsing with Wash Buffer (50 mM Tris-HCl (pH 8.0), 140 mM NaCl, 0.05% Tween 20) 3 times, 200 μl of 5% BSA Wash Buffer was added, followed by incubation at room temperature for 1 hour. After rinsing with Wash Buffer 3 times, 100 μl of a solution of extracellular vesicle in PBS prepared to be 2 μg/ml was added, followed by shaking at room temperature for 2 hours. After rinsing with Wash Buffer 3 times, 100 μl of a 2000-fold diluted HRP-labeled anti-CD9 antibody/anti-CD 63 antibody solution was added as a detection antibody, followed by incubation at room temperature for 1 hour. After rinsing with Wash Buffer 5 times, 100 μl of TMB microwell peroxidase substrate [KPL, 5120-0053] was added, followed by reaction at room temperature for 20 minutes. The reaction was stopped by adding 50 μl of 2% H2SO4, and the absorbance at 450 nm was measured.

Collection of cell culture supernatant

The culture supernatant of KMC07 cultured for 9 days and the culture supernatant of PA6 cultured in KMC Medium for 7 days were collected. First, the collected culture supernatant was centrifuged at 300 g for 5 minutes. The supernatant was then centrifuged at 1,200 g for 20 minutes. Furthermore, the supernatant was centrifuged at 10,000 g for 30 minutes. Finally, the supernatant was collected for use in the subsequent operations.

Biotin labeling of antibody

Biotin Labeling Kit-SH [Dojindo, LK10] was used for biotin labeling of the antibody.

Fluorescence detection sandwich ELISA

In an ELISA plate for fluorescence detection [Greiner, 655077] was seeded 100 μl each of the anti-KMC07 antibody prepared to be 5 μg/ml as a capture antibody and a positive control anti-CD9 antibody 2H12-B11, followed by incubation at 37°C for 1 hour. After rinsing with PBS once, 200 μl of a blocking solution (a 1% solution of BSA in PBS) was added, followed by incubation at room temperature for 30 minutes. After removal of the solution, 100 μl of cell culture supernatant was added, followed by incubation at room temperature for 1 hour. After rinsing with PBS three times, 100 μl of biotin-labeled anti-CD9 antibody 1G10-C11 prepared to be 1 μg/ml was added, followed by incubation at room temperature for 1 hour. After rinsing 3 times with PBS, 50 μl of a streptavidin-β-galactosidase solution [Roche, 11112481001] diluted with a blocking solution to 0.05 U/ml was added, followed by incubation at room temperature for 1 hour. After rinsing with PBS 3 times, 50 μl of a substrate solution (0.2 mM 4-MU [SIGMA, M-1633], 0.2 M sodium phosphate buffer, 1 mM magnesium chloride, 100 mM sodium chloride, 0.4% sodium azide, 0.1% BSA) was added, followed by reaction at 37°C for 2 hours under a light-shielded condition. Finally, 50 μl of a reaction stop solution (0.1 M glycine (pH 10.2)) was added, and then fluorescence at a wavelength of 445 nm was measured at an excitation light wavelength of 372 nm.

4-2. Result

4-2-1. Evaluation of reactivity against KMC07-derived extracellular vesicles by dot blot

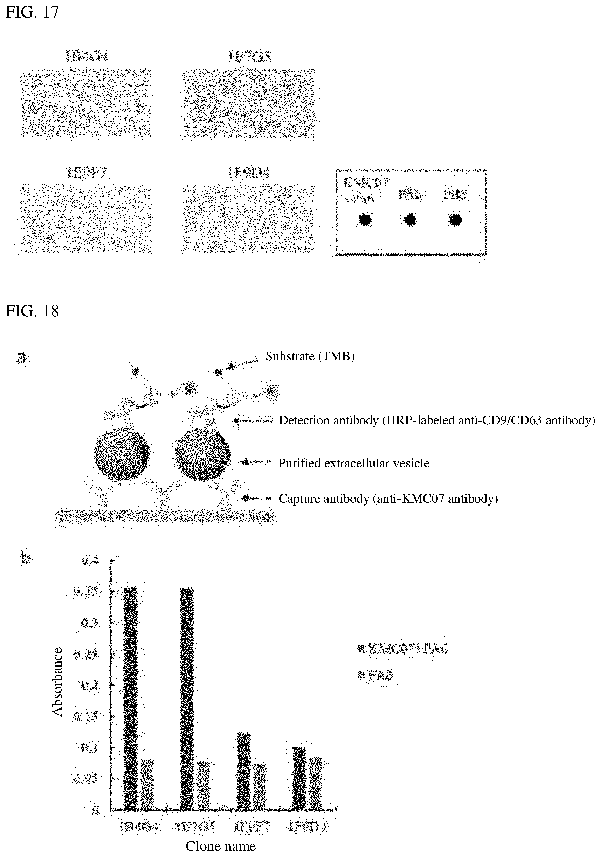

Using extracellular vesicles purified from the culture supernatants of KMC07 + PA6 and PA6, dot blot was performed on 4 clones of the established anti-KMC07 antibody 1B4G4 (antibody I), 1E7G5 (antibody I), 1E9F7 (antibody II), and 1F9D4 (antibody III). As a negative control, PBS was spotted. The results are shown in Fig. 17. As shown in Fig. 17, 1B4G4, 1E7G5, and 1E9F7 showed reactivity only against the KMC07 + PA6-derived exosome. In addition, 1F9D4 showed some reactivity against extracellular vesicles derived from KMC07 + PA6. From this result, it is considered that all the clones strongly recognize proteins contained in KMC07-derived extracellular vesicles.

4-2-2. Evaluation of reactivity against KMC07-derived extracellular vesicles by sandwich ELISA

In order to evaluate the usefulness of the antibody as a diagnostic agent targeting extracellular vesicles, the binding force of each antibody to the extracellular vesicles was examined in a state where the structure of the extracellular vesicles was maintained. Using extracellular vesicles purified from the culture supernatants of KMC07 + PA6 and PA6, sandwich ELISA was performed using 4 clones of the established anti-KMC07 antibody as a capture antibody, and HRP-labeled antibodies against CD9 and CD63, which are extracellular vesicle common markers, as detection antibodies. A schematic diagram of the experimental system is shown in Fig. 18a, and the results are shown in Fig. 18b. As shown in Fig. 18b, 2 clones of 1B4G4 (antibody I) and 1E7G5 (antibody I) exhibited reactivity specifically with KMC07 derived extracellular vesicles, and 1E9F7 (antibody II) and 1F9D4 (antibody III) also slightly reacted specifically with KMC07 derived extracellular vesicles. Therefore, it is considered that these clones can be used as a diagnostic agent specifically targeting extracellular vesicles. In addition, this result correlated with the result of dot blot using extracellular vesicles, and these clones also exhibited reactivity against the KMC07-derived exosome in sandwich ELISA. Therefore, it was suggested that the reactivity against extracellular vesicles maintaining the membrane structure can be evaluated to some extent by dot blot. The dot blot is considered to be very effective as a means for screening antibodies that recognize extracellular vesicles.

4-2-3. Reactivity against KMC07-derived exosome in cell culture supernatant

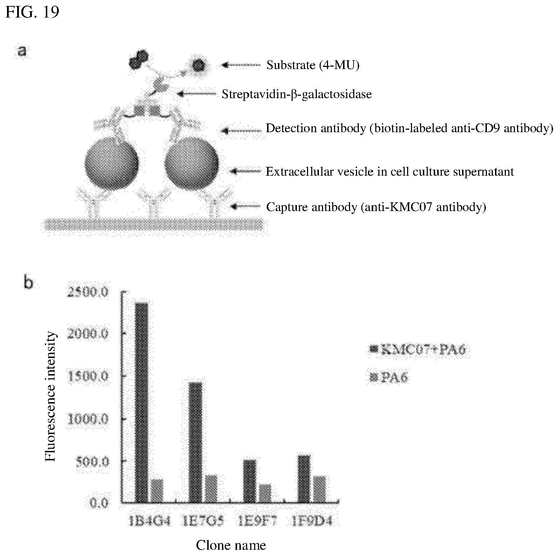

It was aimed to detect a trace amount of extracellular vesicles contained in the cell culture supernatant by a high sensitivity fluorescence detection sandwich ELISA. In a preliminary experiment, it was confirmed that the fluorescence detection sandwich ELISA showed approximately 100 times the sensitivity as compared to color development. Using culture supernatants of KMC07 + PA6 and PA6, 4 clones of the established anti-KMC07 antibody 1B4G4 (antibody I), 1E7G5 (antibody I), 1E9F7 (antibody II), and 1F9D4 (antibody III) were used as a capture antibody, and biotin-labeled anti-CD9 antibody 1G10-C11 was used as a detection antibody for fluorescence detection sandwich ELISA. A schematic diagram of the experimental system is shown in Fig. 19a, and the results are shown in Fig. 19b. As shown in Fig. 19b, 2 clones of 1B4G4 (antibody I) and 1E7G5 (antibody I) specifically reacted against the KMC07 culture supernatant, and 1E9F7 (antibody II) and 1F9D4 (antibody III) also slightly exhibited reactivity against the KMC07 culture supernatant. These results showed the same results as in the color development detection sandwich ELISA, and thus it can be said that extracellular vesicles derived from KMC07 could be detected in the cell culture supernatant by the anti-KMC07 antibody. In addition, it was suggested that the fluorescence detection sandwich ELISA sufficiently functions as a pancreatic cancer diagnostic tool for which detection of a trace amount of extracellular vesicles contained in a sample is required.

Test Example 5: CDR sequencing

CDR sequencing was performed for 1B4G4 (antibody I), 1E7G5 (antibody I), 1E9F7 (antibody II), and 1F9D4 (antibody III). Tables 8A to 8C show the CDRs defined by all combinations of the Kabat numbering program (Bioinformatics, 32, 298-300 (2016)), the IMGT system (Dev Comp Immunol. 27, 55-77 (2003)), and the Paratome algorithm (Nucleic Acids Res. 40 (Web Server issue): W521-4 (2012). doi: 10.1093/nar/gks 480 and PLoS Comput Biol. 8(2): e1002388 (2012)) (Kabat/IMGT/Paratome), the CDRs defined by the Kabat numbering program, the CDRs defined by the IMGT system, and the CDRs defined by the Paratome algorithm.

[Table 8A]

| | | Kabat/IMGT/Paratome | Kabat | IMGT | Paratome |
|---|---|---|---|---|---|
| Antibody I 1B4G4 | HCDR1 | Position 26 to position 35 of SEQ ID NO: 1 (A) | Position 31 to position 35 of SEQ ID NO: 1 (B) | Position 26 to position 33 of SEQ ID NO: 1 (C) | Position 27 to position 35 of SEQ ID NO: 1 (D) |
| | HCDR2 | Position 47 to position 65 of SEQ ID NO: 1 | Position 50 to position 65 of SEQ ID NO: 1 | Position 51 to position 57 of SEQ ID NO: 1 (E) | Position 47 to position 59 of SEQ ID NO: 1 |
| | HCDR3 | Position 96 to position 104 of SEQ ID NO: 1 (F) | Position 98 to position 104 of SEQ ID NO: 1 (G) | Position 96 to position 104 of SEQ ID NO: 1 (F) | Position 97 to position 104 of SEQ ID NO: 1 |
| | LCDR1 | Position 24 to position 34 of SEQ ID NO: 2 (H) | Position 24 to position 34 of SEQ ID NO: 2 (H) | Position 27 to position 32 of SEQ ID NO: 2 | Position 27 to position 34 of SEQ ID NO: 2 |
| | LCDR2 | Position 46 to position 56 of SEQ ID NO: 2 (I) | Position 50 to position 56 of SEQ ID NO: 2 (J) | Position 50 to position 51 of SEQ ID NO: 2 | Position 46 to position 56 of SEQ ID NO: 2 (1) |
| | LCDR3 | Position 89 to position 96 of SEQ ID NO: 2 (K) | Position 89 to position 96 of SEQ ID NO: 2 (K) | Position 89 to position 96 of SEQ ID NO: 2 (K) | Position 89 to position 95 of SEQ ID NO: 2 (L) |
| Antibody I 1E7G5 | HCDR1 | Position 26 to position 35 of SEQ ID NO: 3 (A) | Position 31 to position 35 of SEQ ID NO: 3 (B) | Position 26 to position 33 of SEQ ID NO: 3 (C) | Position 27 to position 35 of SEQ ID NO: 3 (D) |
| | HCDR2 | Position 47 to position 65 of SEQ ID NO: 3 | Position 50 to position 65 of SEQ ID NO: 3 | Position 51 to position 57 of SEQ ID NO: 3 (E) | Position 47 to position 60 of SEQ ID NO: 3 |
| | HCDR3 | Position 96 to position 104 of SEQ ID NO: 3 (F) | Position 98 to position 104 of SEQ ID NO: 3 (G) | Position 96 to position 104 of SEQ ID NO: 3 (F) | Position 96 to position 104 of SEQ ID NO: 3 (F) |
| | LCDR1 | Position 24 to position 34 of SEQ ID NO: 4 (M) | Position 24 to position 34 of SEQ ID NO: 4 (M) | Position 27 to position 32 of SEQ ID NO: 4 | Position 27 to position 34 of SEQ ID NO: 4 |
| | LCDR2 | Position 46 to position 56 of SEQ ID NO: 4 (N) | Position 50 to position 56 of SEQ ID NO: 4 (J) | Position 50 to position 51 of SEQ ID NO: 4 | Position 46 to position 56 of SEQ ID NO: 4 (N) |
| | LCDR3 | Position 89 to position 96 of SEQ ID NO: 4 (K) | Position 89 to position 96 of SEQ ID NO: 4 (K) | Position 89 to position 96 of SEQ ID NO: 4 (K) | Position 89 to position 95 of SEQ ID NO: 4 (L) |
| In table, CDRs bearing the same alphabet (in parenthesis) have identical sequences to each other. | | | | | |

[Table 8B]

| | | Kabat/IMGT/ Paratome | Kabat | IMGT | Paratome |
|---|---|---|---|---|---|
| Antibody II 1E9F7 | HCDR1 | Position 26 to position 35 of SEQ ID NO: 5 | Position 31 to position 35 of SEQ ID NO: 5 | Position 26 to position 33 of SEQ ID NO: 5 | Position 27 to position 35 of SEQ ID NO: 5 |
| | HCDR2 | Position 47 to position 66 of SEQ ID NO: 5 | Position 50 to position 66 of SEQ ID NO: 5 | Position 51 to position 58 of SEQ ID NO: 5 | Position 47 to position 60 of SEQ ID NO: 5 |
| | HCDR3 | Position 97 to position 111 of SEQ ID NO: 5 (O) | Position 99 to position 111 of SEQ ID NO: 5 | Position 97 to position 111 of SEQ ID NO: 5 (O) | Position 98 to position 111 of SEQ ID NO: 5 |
| | LCDR1 | Position 24 to position 34 of SEQ ID NO: 6 (P) | Position 24 to position 34 of SEQ ID NO: 6 (P) | Position 27 to position 32 of SEQ ID NO: 6 | Position 27 to position 34 of SEQ ID NO: 6 |
| | LCDR2 | Position 46 to position 56 of SEQ ID NO: 6 (Q) | Position 50 to position 56 of SEQ ID NO: 6 | Position 50 to position 51 of SEQ ID NO: 6 | Position 46 to position 56 of SEQ ID NO: 6 (Q) |
| | LCDR3 | Position 89 to position 98 of SEQ ID NO: 6 (R) | Position 89 to position 98 of SEQ ID NO: 6 (R) | Position 89 to position 98 of SEQ ID NO: 6 (R) | Position 89 to position 97 of SEQ ID NO: 6 |
| In table, CDRs bearing the same alphabet (in parenthesis) have identical sequences to each other. | | | | | |

[Table 8C]

| | | Kabat/IMGT/ Paratome | Kabat | IMGT | Paratome |
|---|---|---|---|---|---|
| Antibody III 1F9D4 | HCDR1 | Position 26 to position 35 of SEQ ID NO: 7 | Position 31 to position 35 of SEQ ID NO: 7 | Position 26 to position 33 of SEQ ID NO: 7 | Position 27 to position 35 of SEQ ID NO: 7 |
| | HCDR2 | Position 47 to position 66 of SEQ ID NO: 7 | Position 50 to position 66 of SEQ ID NO: 7 | Position 51 to position 58 of SEQ ID NO: 7 | Position 47 to position 61 of SEQ ID NO: 7 |
| | HCDR3 | Position 97 to position 112 of SEQ ID NO: 7 (S) | Position 99 to position 112 of SEQ ID NO: 7 | Position 97 to position 112 of SEQ ID NO: 7 (S) | Position 98 to position 112 of SEQ ID NO: 7 |
| | LCDR1 | Position 24 to position 34 of SEQ ID NO: 8 (T) | Position 24 to position 34 of SEQ ID NO: 8 (T) | Position 27 to position 32 of SEQ ID NO: 8 | Position 27 to position 34 of SEQ ID NO: 8 |
| | LCDR2 | Position 46 to position 56 of SEQ ID NO: 8 (D) | Position 50 to position 56 of SEQ ID NO: 8 | Position 50 to position 51 of SEQ ID NO: 8 | Position 46 to position 56 of SEQ ID NO: 8 (D) |
| | LCDR3 | Position 89 to position 97 of SEQ ID NO: 8 (V) | Position 89 to position 97 of SEQ ID NO: 8 (V) | Position 89 to position 97 of SEQ ID NO: 8 (V) | Position 89 to position 96 of SEQ ID NO: 8 |
| In table, CDRs bearing the same alphabet (in parenthesis) have identical sequences to each other. | | | | | |

**Claims**

1. An antibody against pancreatic cancer stem cells, comprising a heavy chain variable region including heavy chain CDRs as depicted in HCDR1 to HCDR3 below, and a light chain variable region including light chain CDRs as depicted in LCDR1 to LCDR3 below:

   HCDR1 including an amino acid sequence from position 26 to position 35, from position 31 to position 35, from position 26 to position 33, or from position 27 to position 35 of SEQ ID NO: 1; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,
   HCDR2 including an amino acid sequence from position 47 to position 65, from position 50 to position 65, from position 51 to position 57, or from position 47 to position 59 of SEQ ID NO: 1; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,
   HCDR3 including an amino acid sequence from position 96 to position 104, from position 98 to position 104, or from position 97 to position 104 of SEQ ID NO: 1; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,
   LCDR1 including an amino acid sequence from position 24 to position 34, from position 27 to position 32, or from position 27 to position 34 of SEQ ID NO: 2; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,
   LCDR2 including an amino acid sequence from position 46 to position 56, from position 50 to position 56, or from position 50 to position 51 of SEQ ID NO: 2; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions, and
   LCDR3 including an amino acid sequence from position 89 to position 96 or from position 89 to position 95 of SEQ ID NO: 2; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions.

2. The antibody according to claim 1, wherein the HCDR1 includes an amino acid sequence from position 26 to position 35, from position 31 to position 35, from position 26 to position 33, or from position 27 to position 35 of SEQ ID NO: 1 or SEQ ID NO: 3,

   the HCDR2 includes an amino acid sequence from position 47 to position 65, from position 50 to position 65, from position 51 to position 57, from position 47 to position 59, or from position 47 to position 60 of SEQ ID NO: 1 or SEQ ID NO: 3,
   the HCDR3 includes an amino acid sequence from position 96 to position 104, from position 98 to position 104, or from position 97 to position 104 of SEQ ID NO: 1 or SEQ ID NO: 3,
   the LCDR1 includes an amino acid sequence from position 24 to position 34, from position 27 to position 32, or from position 27 to position 34 of SEQ ID NO: 2 or SEQ ID NO: 4,
   the LCDR2 includes an amino acid sequence from position 46 to position 56, from position 50 to position 56, or from position 50 to position 51 of SEQ ID NO: 2 or SEQ ID NO: 4, and
   the LCDR3 includes an amino acid sequence from position 89 to position 96 or from position 89 to position 95 of SEQ ID NO: 2 or SEQ ID NO: 4.

3. The antibody according to claim 1 or 2, comprising:

a heavy chain variable region including an amino acid sequence of SEQ ID NO: 1; an amino acid sequence having the amino acid sequence of SEQ ID NO: 1 in which one to several amino acids are substituted, deleted, added, and/or inserted; an amino acid sequence having 85% or more sequence identity to the amino acid sequence of SEQ ID NO: 1; or an amino acid sequence encoded by a polynucleotide that specifically hybridizes to a polynucleotide including a base sequence complementary to a base sequence encoding the amino acid sequence of SEQ ID NO: 1 under stringent conditions, and

a light chain variable region including an amino acid sequence of SEQ ID NO: 2; an amino acid sequence having the amino acid sequence of SEQ ID NO: 2 in which one to several amino acids are substituted, deleted, added, and/or inserted; an amino acid sequence having 85% or more sequence identity to the amino acid sequence of SEQ ID NO: 2; or an amino acid sequence encoded by a polynucleotide that specifically hybridizes to a polynucleotide including a base sequence complementary to a base sequence encoding the amino acid sequence of SEQ ID NO: 2 under stringent conditions.

4. The antibody according to claim 3, wherein the heavy chain variable region includes an amino acid sequence of SEQ ID NO: 1 or SEQ ID NO: 3, and the light chain variable region includes an amino acid sequence of SEQ ID NO: 2 or SEQ ID NO: 4.

5. An antibody against pancreatic cancer stem cells, comprising a heavy chain variable region including heavy chain CDRs as depicted in HCDR1 to HCDR3 below, and a light chain variable region including light chain CDRs as depicted in LCDR1 to LCDR3 below:

HCDR1 including an amino acid sequence from position 26 to position 35, from position 31 to position 35, from position 26 to position 33, or from position 27 to position 35 of SEQ ID NO: 5; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,

HCDR2 including an amino acid sequence from position 47 to position 66, from position 50 to position 66, from position 51 to position 58, or from position 47 to position 60 of SEQ ID NO: 5; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,

HCDR3 including an amino acid sequence from position 97 to position 111, from position 99 to position 111, or from position 98 to position 111 of SEQ ID NO: 5; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,

LCDR1 including an amino acid sequence from position 24 to position 34, from position 27 to position 32, or from position 27 to position 34 of SEQ ID NO: 6; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,

LCDR2 including an amino acid sequence from position 46 to position 56, from position 50 to position 56, or from position 50 to position 51 of SEQ ID NO: 6; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions, and

LCDR3 including an amino acid sequence from position 89 to position 98 or from position 89 to position 97 of SEQ ID NO: 6; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions.

6. An anti-EpCAM antibody, comprising a heavy chain variable region including heavy chain CDRs as depicted in HCDR1 to HCDR3 below, and a light chain variable region including light chain CDRs as depicted in LCDR1 to LCDR3 below:

HCDR1 including an amino acid sequence from position 26 to position 35, from position 31 to position 35, from position 26 to position 33, or from position 27 to position 35 of SEQ ID NO: 7; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,
HCDR2 including an amino acid sequence from position 47 to position 66, from position 50 to position 66, from position 51 to position 58, or from position 47 to position 61 of SEQ ID NO: 7; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,
HCDR3 including an amino acid sequence from position 97 to position 112, from position 99 to position 112, or from position 98 to position 112 of SEQ ID NO: 7; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,
LCDR1 including an amino acid sequence from position 24 to position 34, from position 27 to position 32, or from position 27 to position 34 of SEQ ID NO: 8; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions,
LCDR2 including an amino acid sequence from position 46 to position 56, from position 50 to position 56, or from position 50 to position 51 of SEQ ID NO: 8; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions, and
LCDR3 including an amino acid sequence from position 89 to position 97 or from position 89 to position 96 of SEQ ID NO: 8; an amino acid sequence having the amino acid sequence in which one to several amino acids are substituted, deleted, added, and/or inserted; an amino acid sequence having 85% or more sequence identity to the amino acid sequence; or an amino acid sequence encoded by a polynucleotide that specifically hybridizes to a polynucleotide including a base sequence complementary to a base sequence encoding the amino acid sequence under stringent conditions.

7. The antibody according to any one of claims 1, 5, and 6, which is a monoclonal antibody.

8. The antibody according to any one of claims 1, 5, and 6, which is a human chimerized antibody or a mouse chimerized antibody.

9. A DNA encoding the antibody according to any one of claims 1, 5, and 6.

10. A recombinant vector comprising the DNA according to claim 9.

11. The recombinant vector according to claim 10, comprising a light chain signal sequence including a base sequence set forth in SEQ ID NO: 20, 24, or 28.

12. A transformant into which the recombinant vector according to claim 10 has been introduced.

13. A method for producing an antibody, comprising the step of culturing the transformant according to claim 12 to produce the antibody according to any one of claims 1, 5, and 6.

14. A pharmaceutical composition comprising the antibody according to any one of claims 1, 5, and 6 as an active ingredient.

15. The pharmaceutical composition according to claim 14 for use in treatment of pancreatic cancer.

16. An extracorporeal diagnostic agent comprising the antibody according to any one of claims 1, 5, and 6.

17. The extracorporeal diagnostic agent according to claim 16 for use in detection of extracellular vesicles derived from cancer cells.

18. The extracorporeal diagnostic agent according to claim 16 for use in diagnosis of pancreatic cancer.

19. An antibody substrate, comprising the extracorporeal diagnostic agent according to claim 16, and a substrate on which the extracorporeal diagnostic agent is immobilized.

20. An extracorporeal diagnostic composition comprising the extracorporeal diagnostic agent according to claim 16.

FIG. 1

Immunization of rat      Cell fusion      Screening      Antibody purification

FIG. 2

Hybridoma      Extraction of total RNA      Synthesis of cDNA

Confirmation of inserted gene sequence

PCR (amplification of variable region gene)      TA cloning

When confirmed as variable region sequence, incorporating gene sequence into antibody expression vector

Ligation by NE builder      Construction of antibody expression vector

FIG. 3

FIG. 4

Culture supernatant of HEK293T
into which antibody expression
vector has been introduced

Positive control
(purified antibody 1.0 µg/ml)

FIG. 5

pCEC2.1-1E7SP-1F9D4 Light Chain

FIG. 6

Culture supernatant of HEK293T into which
vector for expressing light chain SP sequence
recombinant antibody has been introduced

Positive control
(purified antibody 1.0 µg/ml)

FIG. 7

FIG. 8A

FIG. 8B

① 1B4G4 (original)
② 1E7G5 (original)
③ 1F9D4 (original)
④ 1E7G5 (1B4SP-1E7LC)
⑤ 1F9D4 (1B4SP-1F9LC)

FIG. 9

FIG. 10

① 1B4G4 ② 1E2D3 ③ 1E7G5 ④ 1E9F7 ⑤ 1F9D4 ⑥ Control IgG antibody

FIG. 11

① 1B4G4 ② 1E2D3 ③ 1E9F7 ④ 1E7G5 ⑤ 1F9D4

FIG. 12

FIG. 13

FIG. 14A

a    1B4G4

(*P<0.05. **P<0.001, two-way ANOVA)

FIG. 14B

b 1F9D4

(*P<0.05, **P<0.001, two-way ANOVA)

FIG. 15

NK92/CD16a only     1F9D4-NK92/CD16a

(Scale bars: 200 μm)

FIG. 16A

FIG. 16B

FIG. 17

FIG. 18

a

Substrate (TMB)

Detection antibody (HRP-labeled anti-CD9/CD63 antibody)

Purified extracellular vesicle

Capture antibody (anti-KMC07 antibody)

b

Absorbance

Clone name

*KMC07+PA6
*PA6

FIG. 19

a

- ← Substrate (4-MU)
- ← Streptavidin-β-galactosidase
- ← Detection antibody (biotin-labeled anti-CD9 antibody)
- ← Extracellular vesicle in cell culture supernatant
- ← Capture antibody (anti-KMC07 antibody)

b

■ KMC07+PA6

■ PA6

<div align="center">**INTERNATIONAL SEARCH REPORT**</div>

| | International application No. |
|---|---|
| | **PCT/JP2022/017037** |

**A. CLASSIFICATION OF SUBJECT MATTER**

*C12N 15/13*(2006.01)i; *A61K 39/395*(2006.01)i; *A61P 1/18*(2006.01)i; *A61P 35/00*(2006.01)i; *A61P 43/00*(2006.01)i; *C07K 16/28*(2006.01)i; *C07K 16/32*(2006.01)i; *C07K 16/46*(2006.01)i; *C12N 1/15*(2006.01)i; *C12N 1/19*(2006.01)i; *C12N 1/21*(2006.01)i; *C12N 5/10*(2006.01)i; *C12N 15/63*(2006.01)i; *C12P 21/08*(2006.01)i; *G01N 33/574*(2006.01)i
FI: C12N15/13; A61P1/18; A61P35/00; A61P43/00 105; C07K16/32; C07K16/28; C12N15/63 Z; C12N1/15; C12N1/19; C12N1/21; C12N5/10; C12P21/08; C07K16/46; G01N33/574 D; A61K39/395 N ZNA

According to International Patent Classification (IPC) or to both national classification and IPC

**B. FIELDS SEARCHED**

Minimum documentation searched (classification system followed by classification symbols)

C12N15/13; A61K39/395; A61P1/18; A61P35/00; A61P43/00; C07K16/28; C07K16/32; C07K16/46; C12N1/15; C12N1/19; C12N1/21; C12N5/10; C12N15/63; C12P21/08; G01N33/574

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq; UniProt/GeneSeq

**C. DOCUMENTS CONSIDERED TO BE RELEVANT**

| Category* | Citation of document, with indication, where appropriate, of the relevant passages | Relevant to claim No. |
|---|---|---|
| Y | SUN, L. et al. Screen and identification of monoclonal antibody against pancreatic cancer stem cells. Journal of Modern Oncology. 2014, vol. 22, no. 7, pp. 1492-1496 <br> abstract, pp. 1493-1494 | 1-20 |
| X | JP 2020-524527 A (SUZHOU BOJUHUA BIO MEDICAL TECHNOLOGY CO., LTD.) 20 August 2020 (2020-08-20) <br> entirety in particular, claims, examples | 1-20 |
| Y | | 1-20 |
| X | CIOFFI, M. et al. EpCAM/CD3-Bispecific T-cell Engaging Antibody MT110 Eliminates Primary Human Pancreatic Cancer Stem Cells. Clinical Cancer Research. 2011, vol. 18, no. 2, pp. 465-474 <br> abstract, p. 466, left column | 6-20 |
| A | | 1-5 |

☑ Further documents are listed in the continuation of Box C.     ☑ See patent family annex.

| | |
|---|---|
| *   Special categories of cited documents: <br> "A"   document defining the general state of the art which is not considered to be of particular relevance <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **14 June 2022** | **28 June 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**

| International application No. |
|---|
| **PCT/JP2022/017037** |

**C.     DOCUMENTS CONSIDERED TO BE RELEVANT**

| Category* | Citation of document, with indication, where appropriate, of the relevant passages | Relevant to claim No. |
|---|---|---|
| X | US 2012/0095192 A1 (TRION PHARMA GMBH) 19 April 2012 (2012-04-19)<br>      example 1 | 6-20 |
| A | | 1-5 |
| A | KR 10-2020-0132110 A (KNU INDUSTRY COOPERATION FOUNDATION) 25 November 2020 (2020-11-25)<br>      claims, examples | 1-20 |

**INTERNATIONAL SEARCH REPORT**

International application No.

**PCT/JP2022/017037**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed:

          ☑  in the form of an Annex C/ST.25 text file.

          ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

          ☐  in the form of an Annex C/ST.25 text file (Rule 13*ter*.1(a)).

          ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**
**Information on patent family members**

| | International application No. |
|---|---|
| | **PCT/JP2022/017037** |

| Patent document cited in search report | Publication date (day/month/year) | Patent family member(s) | Publication date (day/month/year) |
|---|---|---|---|
| JP 2020-524527 A | 20 August 2020 | US 2021/0147571 A1 claims, examples<br>WO 2018/233333 A1<br>EP 3650469 A1<br>CN 107118276 A | |
| US 2012/0095192 A1 | 19 April 2012 | WO 2010/119119 A1<br>EP 2241576 A1 | |
| KR 10-2020-0132110 A | 25 November 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005080432 A
- US 4816567 A


**Non-patent literature cited in the description**

- **BORST P.** Cancer drug pan-resistance: pumps, cancer stem cells, quiescence, epithelial to mesenchymal transition, blocked cell death pathways, persisters or what?. *Open Biol.,* 2012, vol. 2, 120066
- **HOLOHAN C. ; VAN SCHAEYBROECK, S. ; LONGLEY, D. B. ; JOHNSTON, P.G.** Cancer drug resistance: an evolving paradigm. *Nat. Rev. Cancer.,* 2013, vol. 13, 714-726
- **DIEHN M ; CHO RW ; LOBO NA ; KALISKY T ; DORIE MJ et al.** Association of reactive oxygen species levels and radioresistance in cancer stem cells. *Nature,* 2009, vol. 458, 780-783
- **XIAOXIAN LI ; MICHAEL T. LEWIS ; JIAN HUANG ; CAROLINA GUTIERREZ ; C. KENT OSBORNE et al.** Intrinsic resistance of tumorigenic breast cancer cells to chemotherapy. *J. Natl. Cancer Inst.,* 2008, vol. 100, 672-679
- **SHIDENG BAO ; QIULIAN WU1 ; ROGER E. MCLENDON ; YUELING HAO ; QING SHI et al.** Glioma stem cells promote radioresistance by preferential activation of the DNA damage response. *Nature,* 2006, vol. 444, 756-760
- **EDUARD BATLLE ; HANS CLEVERS.** Cancer stem cells revisited. *Nat. Med.,* 2017, vol. 23, 1124-1134
- **NAOKO TAKEBE ; LUCIO MIELE ; PAMELA JO HARRIS ; WOONDONG JEONG ; HIDEAKI BANDO et al.** Targeting Notch, Hedgehog, and Wnt pathways in cancer stem cells: clinical update. *Nat. Rev. Clin. Oncol.,* 2015, vol. 12, 445-464
- **LASZLO G.S. ; ESTEY E.H. ; WALTER R.B.** The past and future of CD33 as therapeutic target in acute myeloid leukemia. *Blood Rev.,* 2014, vol. 28, 143-153
- *Bioinformatics,* 2016, vol. 32, 298-300
- *Dev Comp Immunol.,* 2003, vol. 27, 55-77
- *Nucleic Acids Res.,* 2012, vol. 40, W521-4
- *PLoS Comput Biol.,* 2012, vol. 8 (2), e 1002388
- **HASHIMOTO-GOTOH T. et al.** *Gene,* 1995, vol. 152, 271-275
- **ZOLLER MJ. et al.** *Methods Enzymol.,* 1983, vol. 100, 468-500
- **KRAMER W. et al.** *Nucleic Acids Res.,* 1984, vol. 12, 9441-9456
- **KRAMER W. et al.** *Methods. Enzymol.,* 1987, vol. 154, 350-367
- **KUNKEL TA.** *Proc Natl Acad Sci USA.,* 1985, vol. 82, 488-492
- **TATIANA A. TATSUSOVA ; THOMAS L. MADDEN.** *FEMS Microbiol. Lett.,* 1999, vol. 174, 247-250
- **KOHLER G ; MILSTEIN C.** *Nature,* 07 August 1975, vol. 256 (5517), 495-497
- **CLACKSON et al.** *Nature,* 15 August 1991, vol. 352 (6336), 624-628
- **MARKS et al.** *J Mol Biol.,* 05 December 1991, vol. 222 (3), 581-597
- Protein Experiment Handbook. YODOSHA CO., LTD, 2003, 92-96
- **SATO, K. et al.** *Cancer Research,* 1993, vol. 53, 851-856
- **JONES et al.** *Nature,* 1986, vol. 321, 522-525
- **RIECHMANN et al.** *Nature,* 1988, vol. 332, 323-327
- **VERHOEYEN et al.** *Science,* 1988, vol. 239, 1534-1536
- **SHIMIZU K. ; NISHIYAMA T. ; HORI Y.** Gemcitabine Enhances Kras-MEK-Induced Matrix Metalloproteinase-10 Expression Via Histone Acetylation in Gemcitabine-Resistant Pancreatic Tumor-initiating Cells. *Pancreas.,* 2017, vol. 46, 268-275
- **CHRISTINE M F. ; CHARLOTTE K.** Human breast cancer cell lines contain stem-like cells that self-renew, give rise to phenotypically diverse progeny and survive chemotherapy. *Breast Cancer Research,* 2008, vol. 10, R 25
- *PLoS Comput Biol.,* 2012, vol. 8 (2), e1002388